(19) Europäisches Patentamt
European Patent Office
Office européen des brevets

(11) Publication number: **0 361 365 A1**

# (12) EUROPEAN PATENT APPLICATION

(21) Application number: 89117683.6

(22) Date of filing: 25.09.89

(51) Int. Cl.5: C07C 323/60 , C07C 327/32 , A61K 31/195

(30) Priority: 30.09.88 US 251638

(43) Date of publication of application: 04.04.90 Bulletin 90/14

(84) Designated Contracting States: DE FR GB IT

(71) Applicant: E.R. SQUIBB & SONS, INC.
P.O.Box 4000
Princeton New Jersey 08543-4000(US)

(72) Inventor: Delaney, Norma Gail
137 Cherry Brook Drive
Princeton New Jersey 08540(US)

(74) Representative: Staub, Gabriella, Dr.-Ing. et al
Baaderstrasse 3
D-8000 München 5(DE)

## (54) Aminobenzoic and aminocyclohexane-carboylic acid compounds, compositions, and their method of use.

(57) Compounds of the formula

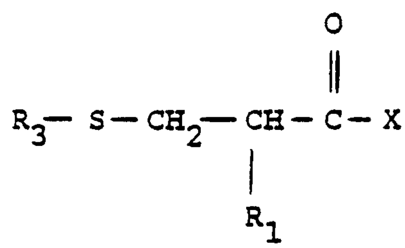

wherein X is 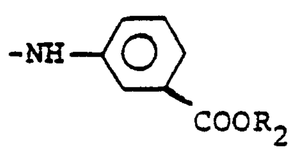 , 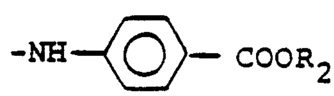 ,

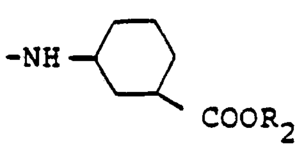 , or 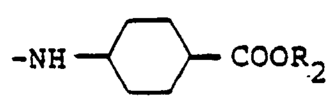

inhibit the action of neutral endopeptidase. As a result, such compounds produce diuresis, natriuresis, and lower blood pressure as well as being useful in the treatment of congestive heart failure, relieving pain, and diarrhea when administered to a mammalian host.

EP 0 361 365 A1

# AMINOBENZOIC AND AMINOCYCLOHEXANECARBOYLIC ACID COMPOUNDS, COMPOSITIONS, AND THEIR METHOD OF USE

This invention is directed to reducing blood pressure and producing diuresis and natriuresis, as well as treating congestive heart failure, pain, and/or diarrhea by administering a pharmaceutical composition containing a neutral endopeptidase inhibitor of formula I and salts thereof

(I)

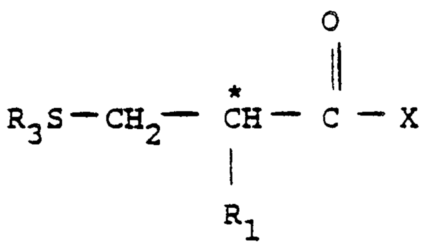

X is

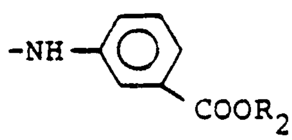 , 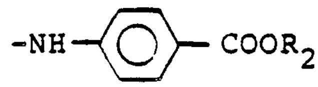 ,

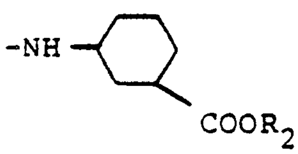 , or 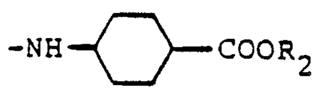 .

$R_1$ is hydrogen, lower alkyl, halo substituted lower alkyl,

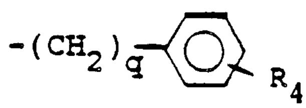 ,

$-(CH_2)_r$-cycloalkyl, $-(CH_2)_r$-($\alpha$-napthyl), $-(CH_2)_r$-($\beta$-naphthyl),

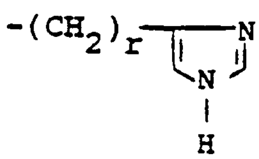 , 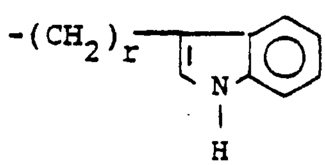 ,

$-(CH_2)_r-NH_2$ , $-(CH_2)_r-SH$ , $-(CH_2)_r$-S-lower alkyl, $-(CH_2)_r-OH$,

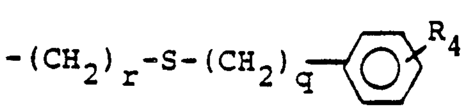 ,

$-(CH_2)_r$-O-lower alkyl,

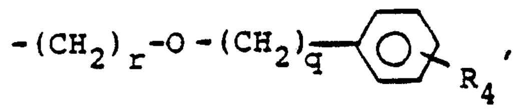

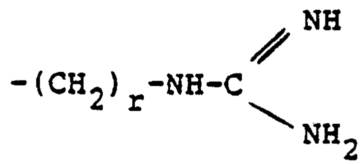,

or

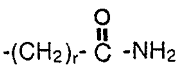 .

$R_2$ is hydrogen, lower alkyl, benzyl, benzhydryl, a salt forming ion, or

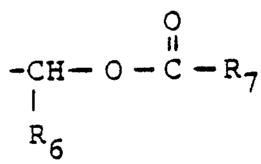 .

$R_3$ is hydrogen or 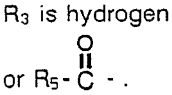 .

$R_5$ is lower alkyl,

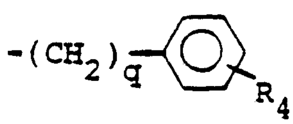,

$-(CH_2)_q$-($\alpha$-naphthyl), $-(CH_2)_q$-($\beta$-naphthyl),

$-(CH_2)_q$-cycloalkyl, 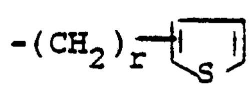,

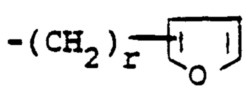, 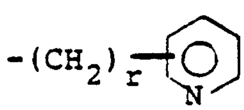,

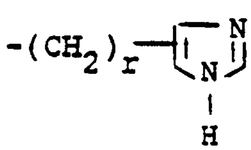, or 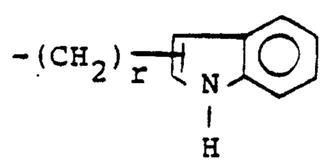 .

$R_4$ is hydrogen, lower alkyl of 1 to 4 carbons, lower alkoxy of 1 to 4 carbons, lower alkylthio of 1 to 4 carbons, halo, hydroxy, $CF_3$, phenyl,

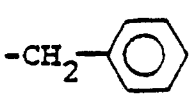, or 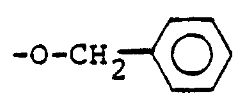 .

$R_6$ is hydrogen, lower alkyl, cycloalkyl, or phenyl.

$R_7$ is hydrogen, lower alkyl, lower alkoxy, or phenyl.

r is an integer from 1 to 4.

q is zero or an integer from 1 to 7.

This invention is also directed to the novel compounds of formula I wherein X is

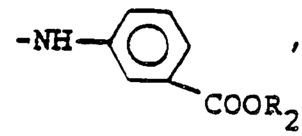 ,

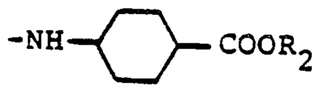 , or 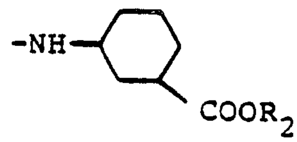

and $R_1$, $R_2$ and $R_3$ are as defined above and the novel compounds of formula I wherein X is

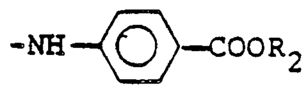

and $R_1$,$R_2$, and $R_3$ are as defined above except that $R_1$ is not methyl.

This invention in its broadest aspects relates to the method of lowering blood pressure and producing diuresis and natriuresis by administering a pharmaceutical composition containing a neutral endopeptidase inhibitor of formula I. This invention is also directed to the novel compounds of formula I wherein $R_1$ is other than methyl or when $R_1$ is methyl, X is 3-aminobenzoic acid, 3- or 4-aminocyclohexanecarboxylic acid.

The term lower alkyl used in defining various symbols refers to straight or branched chain radicals having up to seven carbons. The preferred lower alkyl groups are straight or branched chain of up to four carbons. Similarly the terms lower alkoxy and lower alkylthio refer to such lower alkyl groups attached to an oxygen or sulfur.

The term cycloalkyl refers to saturated rings of 4 to 7 carbons atoms with cyclopentyl and cyclohexyl being most preferred.

The term halogen refers to chloro, bromo , fluoro, and iodo.

The term halo substituted lower alkyl refers to such lower alkyl groups described above in which one or more hydrogens have been replaced by chloro, bromo or fluoro groups such as trifluoromethyl, which is preferred, pentafluoroethyl, 2,2,2-trichloroethyl, chloromethyl, bromomethyl, etc.

The symbols

; 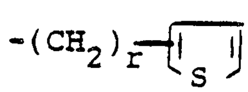 , 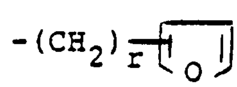 ,

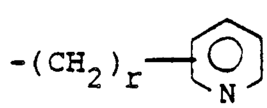 ,

etc., represent that the alkylene bridge is attached to an available carbon atom.

The compounds of formula I can be prepared by coupling an acylthio carboxylic acid of the formula

(II)

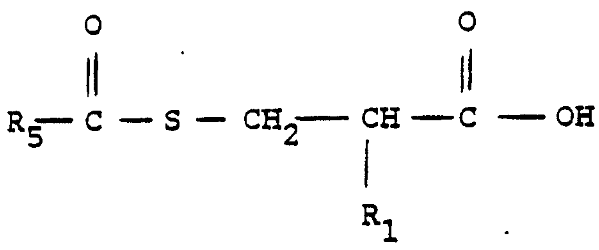

to the aminobenzoic acid ester or aminocyclohexanecarboxylic acid ester of the formula (III) HX .

The aminobenzoic acid ester or aminocyclohexanecarboxylic acid ester of formula III can be employed as the hydrochloride salt and methyl is the preferred ester group. The acylthio carboxylic acid of formula II is preferably converted to an activated form such as an acid chloride, mixed anhydride etc. The reaction is preferably carried out in the presence of diisopropylethylamine.

The resulting acylthio aminobenzoic or aminocyclohexanecarboxylic acid ester can be hydrolyzed by treating with a base such as sodium hydroxide to remove the acyl group and the $R_2$ ester group and yield the desired mercaptan products of formula I, i.e., $R_2$ and $R_3$ are both hydrogen.

Alternatively, the aminobenzoic or aminocyclohexanecarboxylic acid of formula III, i.e., $R_2$ is hydrogen, can be coupled directly to the activated form of the carboxylic acid of formula II by first treating the aminobenzoic or aminocyclohexanecarboxylic acid with bis(trimethylsilyl) trifluoroacetamide. The resulting acylthio aminobenzoic or aminocyclohexanecarboxylic acid can be treated with ammonia to remove the acyl group and yield the desired mercaptan products of formula I, i.e., $R_3$ is hydrogen.

Of course, the mercaptan products of formula I can be acylated with an acid chloride of the formula

(IV) 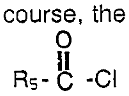

to introduce other acyl groups.

The acylthio carboxylic acids of formula II are described in various literature and patent references. For example, the carboxylic acids wherein $R_1$ is hydrogen, lower alkyl, phenyl, or phenyl-lower alkyl are described by Ondetti et al. in U.S. Patent 4,105,776, the carboxylic acids wherein $R_1$ is alkylthioalkylene are described by Ondetti et al. in U.S. Patent 4,116,962, the carboxylic acids wherein $R_1$ is carbamoylalkylene are described by Ondetti in U.S. Patent 4,091,024, the carboxylic acids wherein $R_1$ is aminoalkylene or guanidinylalkylene are described by Ondetti et al. in U.S. Patent 4,113,715, the carboxylic acids wherein $R_1$ is trifluoromethyl are described by Ondetti et al. in U.S. Patent 4,154,935, etc.

In the above reactions if $R_1$ and/or $R_5$ is

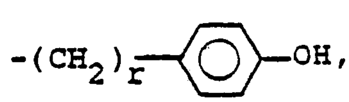, 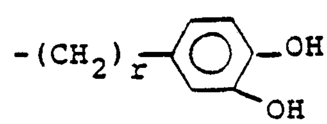, $-(CH_2)_r-NH_2$ ,

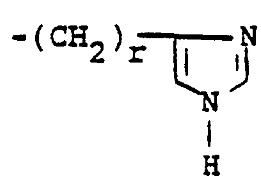, $-(CH_2)_r-SH$ , $(CH_2)_r-OH$ , or

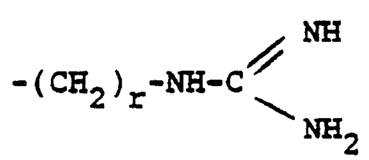

then the hydroxyl, amino imidazolyl, mercaptan, or guanidinyl function should be protected during the coupling reaction. Suitable protecting groups include benzyloxycarbonyl, t-butoxycarbonyl, benzyl, benzhydryl, trityl, etc., and nitro in the case of guanidinyl. The protecting group is removed by treatment with

acid or other known methods following completion of the reaction.

The ester products of formula I wherein $R_2$ is

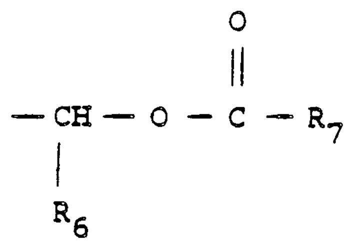

can be obtained by treating the product of formula I wherein $R_2$ is hydrogen with a molar equivalent of a compound of the formula

(V)

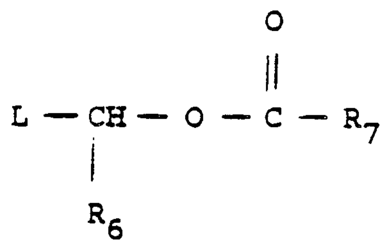

wherein L is a leaving group such as chlorine, bromine, toluenesulfonyloxy, etc., in the presence of base.

Preferred compounds of this invention are those of formula I wherein

$R_1$ is straight or branched chain alkyl of 2 to 4 carbons,

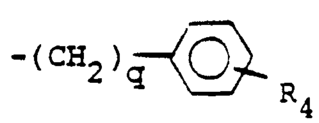

wherein q is zero or an integer from 1 to 4, or trifluoromethyl.

$R_2$ is hydrogen or an alkali metal salt ion.

$R_3$ is hydrogen or

$R_5-\overset{O}{\overset{\|}{C}}-$, especially hydrogen.

$R_4$ is hydrogen or

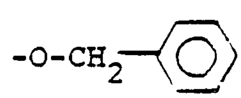

$R_5$ is methyl or phenyl, especially methyl.

Also, preferred as intermediates are the compounds of formula I wherein

$R_1$ is as defined above.

$R_2$ is methyl

$R_3$ is

$R_5-\overset{O}{\overset{\|}{C}}-$.

$R_5$ is methyl or phenyl, especially methyl.

$R_4$ is as defined above.

The compounds of formula I wherein $R_2$ is hydrogen form salts with a variety of inorganic or organic bases. The nontoxic, pharmaceutically acceptable salts are preferred, although other salts are also useful in

isolating or purifying the product. Such pharmaceutically acceptable salts include alkali metal salts such as sodium, potassium or lithium, alkaline earth metal salts such as calcium or magnesium, and salts derived from amino acids such as arginine, lysine, etc. The salts are obtained by reacting the acid form of the compound with an equivalent of the base supplying the desired ion in a medium in which the salt precipitates or in aqueous medium and then lyophilizing.

As shown above, the compounds of formula I wherein $R_1$ is other than hydrogen contain asymmetric centers as represented by the * in formula I. An additional asymmetric center is present in the ester products when $R_6$ is other than hydrogen. Thus, the compounds of formula I can exist in diastereomeric forms or in mixtures thereof. The above described processes can utilize racemates, enantiomers or diastereomers as starting materials. When diastereomeric products are prepared, they can be separated by conventional chromatographic or fractional crystallization methods.

Human as well as other mammalian atria contain specific granules which have been found to contain a precursor to a family of peptides collectively called atrial natriuretic factor (deBold, Science, Vol. 230, p. 767-770, 1985). The biologically active segments of this precursor which circulate in the blood are 21-28 amino acid peptides called atrial natriuretic peptides. These peptides cause diuresis, natriuresis, and relaxation of smooth muscle in blood vessels and other tissues (Needleman et al., Hypertension, Vol. 7, p. 469 - 482, 1985). The putative circulating hormone in man is a 28 amino acid peptide called human ANF 99 - 126. Exogeneous administration of this peptide to man has been reported to cause diuresis, natriuresis, and a fall in blood pressure(Richards et al., Hypertension, Vol. 7, p. 812 - 817, 1985).

The compounds of formula I inhibit the activity of neutral endopeptidase (EC 3.4.24.11), a membrane-bound zinc metallopeptidase found in many tissues including the brain and kidney. Neutral endopeptidase hydrolyzes peptide bonds which are on the amino terminal side of hydrophobic amino acid residues. Atrial natriuretic peptides have been shown to be cleaved at the $Cys^{105}$-$Phe^{106}$ bond by the action of neutral endopeptidase (Delaney et al., Fed. Proc. 46, p. 1296, 1987; Stephenson et al. Biochem. J., Vol. 243, p. 183 - 187, 1987). Cleavage of rat ANF 103 - 126 at $Cys^{105}$-$Phe^{106}$ results in diminishing of its vasorelaxant (Bergey et al. Fed. Proc.46, p. 1296, 1987) and natriuretic, diuretic and depressor activities (Seymour et al., Fed.Proc.46, p. 1296, 1987). Stephensen et al. reported that the hydrolysis of human ANF 99 - 126 by pig kidney microvillar membranes in vitro was suppressed by the neutral endopeptidase inhibitor, phosphoramidon.

While not limiting the scope of this invention to a specific theory or mechanism of action, inhibition of neutral endopeptidase is believed to result in reduced inactivation of exogenously administered or endogenous atrial natriuretic peptides. Thus the compounds of formula I are useful in the treatment of hypertension, congestive heart failure, renal failure or hepatic cirrhosis. Diuresis, natriuresis, and blood pressure reduction are produced in a mammalian host such as man by the administration of from about 1 mg. to about 100 mg. per kg. of body weight per day, preferably from about 1 mg to about 50 mg. per kg. of body weight per day, of one or more neutral endopeptidase inhibitors of formula I or a pharmaceutically acceptable salt thereof. The neutral endopeptidase inhibitors of formula I are preferably administered orally, but parenteral routes such as subcutaneous, intramuscular, and intravenous can also be employed. The daily dose can be administered singly or can be divided into two to four doses administered throughout the day.

The neutral endopeptidase inhibitors of formula I can also be administered in combination with other blood pressure lowering agents. For example, the neutral endopeptidase inhibitors of formula I can be combined for dual administration with an angiotensin converting enzyme (ACE) inhibitor such as captopril, zofenopril, fosinopril, enalapril, lisinopril, etc. Such combination would be at a weight ratio of endopeptidase inhibitor to ACE inhibitor of from about 1:10 to about 10:1.

The neutral endopeptidase inhibitors of formula I can also be administered in combination with human ANF 99 - 126. Such combination would contain the inhibitor of formula I at from about 1 to about 100 mg. per kg. of body weight and the human ANF 99 - 126 at from about 0.001 to about 0.1 mg. per kg. of body weight.

The neutral endopeptidase inhibitors of formula I or pharmaceutically acceptable salts thereof can also be administered to a mammalian host such as man to inhibit the degradation of endogenous opioid pentapeptides, [$Met^5$]-enkephalin (Try-Gly-Gly-Phe-Met) and [$Leu^5$]-enkephalin (Try-Gly-Gly-Phe-Leu), in the brain or in peripheral tissues. Due to its role in the degradation of enkephalinase, brain endopeptidase has often been referred to as "enkephalinase." Enkephalins are neurotransmitters which decrease the perception of pain (Hughes, et al., Nature, Vol. 258, December 1975, p. 577 - 579). These endogenous opioid peptides are functionally inactivated by cleavage of their $Gly^3$-$Phe^4$ peptide bonds by neutral endopeptidase located at nerve terminals in the brain where enkephalins are released (Malfroy, et al., Nature, Vol. 276, November 1978, p. 523 - 526). Neutral endopeptidase inhibitors enhance the recovery of

endogenous enkephalins released from isolated brain slices (Patey, et al., Science, Vol. 212, June 1981, p. 1153 - 1155) and cause analgesia in mice that is reversed by the opiate antagonist naloxone (Roques, et al., Nature, Vol. 288, November 1980, p. 286 - 288). Inhibitors of neutral endopeptidase also show naloxone -reversible antidiarrheal effects in rats (Marcais - Collado, et al., European Journal of Pharmacology, Vol. 144, p. 125 - 132, 1987).

Thus, the compounds of formula I or a pharmaceutically acceptable salt thereof can be administered as an analgesic or antidiarrheal agent to patients orally or parenterally in an effective amount within the daily dosage range of from about 0.1 to about 25 mg. of compound per kg. of patient body weight. Administration can be once daily or in 2 to 4 divided daily doses.

The inhibitors of formula I and other pharmaceutically acceptable ingredients can be formulated for the above described pharmaceutical uses. Suitable compositions for oral administration include tablets, capsules, and elixirs, and suitable compositions for parenteral administration include sterile solutions and suspensions. About 10 to 500 mg. of active ingredient is compounded with physiologically acceptable vehicle, carrier, excipient, binder, preservative, stabilizer, flavoring, etc., in a unit dose form as called for by accepted pharmaceutical practice.

The following examples are illustrative of the invention. Temperatures are given in degrees centigrade.

## Example 1

### 4-[[2-(Mercaptomethyl)-1-oxo-3-phenylpropyl]amino]benzoic acid

#### a) 4-Aminobenzoic acid, methyl ester, hydrochloride

Thionyl chloride (5.84 ml., 80 mmole) was added, dropwise with stirring, to a cold suspension of 4-aminobenzoic acid (5.49 g., 40 mmole) in methanol (100 ml.). The addition was done at a rate so as to maintain the temperature between -5° and -10°. After the addition was completed, the mixture was allowed to warm to room temperature and stirred overnight. The mixture was then concentrated *in vacuo* to give a white solid which was twice triturated in ether to yield 7.25 g. of 4-aminobenzoic acid, methyl ester, hydrochloride as a white solid; m.p. 189 - 192°. TLC (silica gel; n-butanol:acetic acid:water, 4:1:1) $R_f$ = 0.77.

#### b) 3-Acetylthio-2-benzylpropanoic acid

Benzyl malonic acid (13 g., 67 mmole) was mixed with 40% aqueous dimethylamine (7.6 g., 68 mmole) and 37% formalin (5.4 g., 68 mmole) in water (150 ml.). The voluminous solid that formed in 15 minutes was filtered after 2 hours, washed with water, and dried partially in air to give 20.8 g. of solid. The solid was melted in an oil bath (170°) and heated for 10 minutes until amine evolution stopped and bubbling had virtually ceased. The cooled product, a mobile liquid, was acidified with 10% potassium bisulfate, extracted with hexane, dried ($Na_2SO_4$), and evaporated to give 6.3 g. of solid. The aqueous filtrates were allowed to stand overnight and were then heated at 100° on a steam cone until bubbling ceased (2 hours). Cooling, acidification, and extraction gave another 1.2 g. of solid for a total of 7.5 g. of benzylacrylic acid.

A solution of benzylacrylic acid (5.96 g., 40 mmole) in thiolacetic acid (10 ml.) was stirred for 1 hour at room temperature and then heated on a steam bath for one hour. The thiolacetic acid was removed by vacuum distillation, and the resulting dark yellow oil was poured into saturated sodium bicarbonate (much bubbling) and extracted with ethyl acetate (2 x 40 ml.). The aqueous portion was acidified to a pH of about 3 with 10% potassium bisulfate, and reextracted with ethyl acetate (3 x 40 ml.). These extracts were combined, dried ($Na_2SO_4$), and concentrated *in vacuo* to give 6.44 g. of yellow oil 3-acetylthio-2-benzylpropanoic acid.

#### c) 4-[[2-[(Acetylthio)methyl]-1-oxo-3-phenylpropyl]amino]benzoic acid, methyl ester

Oxalyl chloride (0.68 ml., 7.8 mmole) was added to a solution of 3-acetylthio-2-benzylpropanoic acid

(1.79 g., 7.5 mmole) in ether (15 ml.). This mixture was cautiously treated with a catalytic amount (2 drops) of dimethyl formamide, and then stirred at room temperature for 1 hour. The mixture was concentrated in vacuo, producing an oil which was dissolved in tetrahydrofuran (15 ml.) and again concentrated in vacuo. The resulting residue was disssolved in methylene chloride (20 ml.) and added dropwise over 10 minutes to a cold (-5°), stirred suspension of 4-aminobenzoic acid, methyl ester, hydrochloride (1.52 g., 8.1 mmole) and diisopropylethylamine (2.94 ml., 16.9 mmole) in dichloromethane (20 ml.). After stirring in the cold (-5°C) for 2.5 hours, the mixture was allowed to warm to room temperature and allowed to stir overnight. The mixture was concentrated in vacuo and the residue was taken up into ethyl acetate (100 ml.) and filtered to remove diisopropylethylamine, hydrochloride. The filtrate was washed sequentially with 10% potassium bisulfate, water, 5% sodium bicarbonate, water, and 50% brine (3 x 30 ml. each). The organic layer was dried ($Na_2SO_4$) and concentrated to yield 3.0 g. of a yellow oil. This oil was applied to a column of 300 g. of Merck silica gel (230 - 400 mesh) and eluted with hexane/ethyl acetate (2:1) to give 1.13 g. of 4-[[2-[(acetylthio)methyl]-1-oxo-3-phenylpropyl]amino]benzoic acid, methyl ester as an off-white solid; m.p. 97 - 99° (sinters at greater than 92°). TLC (silica gel; ethyl acetate:hexane, 1:1) $R_f$ = 0.49.

d) 4-[[2-(Mercaptomethyl)-1-oxo-3-phenylpropyl]amino]benzoic acid

The methyl ester product from part (c) (1.13 g., 3.04 mmole) was dissolved in methanol (20 ml.) and then chilled in an ice bath under nitrogen. 1N Sodium hydroxide (9.5 ml., 3 equiv.) was added dropwise to this solution over 10 minutes. The mixture was stirred at 0° for 10 minutes and then allowed to warm to room temperature and stirred for 3 hours. The mixture was concentrated in vacuo to remove the methanol. The residue (a white suspension) was diluted with water (40 ml.) and extracted with chloroform (2 x 15 ml.). The organic layer was concentrated in vacuo and the residue was taken up in 1N sodium hydroxide (40 ml.) and extracted with chloroform (2 x 15 ml.). Both aqueous extracts were combined and acidified to a pH of about 1.5 with concentrated HCl. The resulting white suspension was extracted with ethyl acetate (3 x 40 ml.). These extracts were combined, washed with water and brine (3 x 40 ml. each), dried ($Na_2SO_4$), and concentrated to give 910 mg. of an off-white solid. Recrystallization from chloroform yields a solid that was dissolved in methanol and filtered through a cellulose micro-filter to yield 580 mg. of 4-[[2-(mercaptomethyl)-1-oxo-3-phenylpropyl]amino]benzoic acid as an off-white solid; m.p. 178-179°. TLC (silica gel; benzene:acetic acid, 4:1) $R_f$ = 0.43 (trace at 0.36).

| Anal. calc'd. for $C_{17}H_{17}NO_3S$: | | | | |
|---|---|---|---|---|
| | C, 64.74; | H, 5.43; | N, 4.44; | S, 10.17 |
| Found: | C, 64.65; | H, 5.50; | N, 4.47; | S, 10.17. |

## Example 2

(S)-4-[(3-Mercapto-2-methyl-1-oxopropyl)amino]benzoic acid

a) (S)-4-[[3-Acetylthio)-2-methyl-1-oxopropyl]amino]benzoic acid, methyl ester

A suspension of 4-aminobenzoic acid, methyl ester, hydrochloride (6.75 g., 36.0 mmole) and diisopropylethylamine (9.5 g., 73.5 mmole) in dry methylene chloride (80 ml.) was cooled to -10° under nitrogen and treated dropwise with a solution of (D)-3-(acetylthio)-2-methylpropanoyl chloride (6.0 g., 32.7 mmole) in dry methylene chloride (80 ml.). After the addition was completed, the mixture was stirred cold for an additional 2.5 hours and then allowed to warm to ambient temperature overnight. The reaction mixture was concentrated in vacuo. The residue was dissolved in ethyl acetate (300 ml.) and the solution was washed with 50 ml. portions of 10% aqueous potassium bisulfate, water, 5% aqueous sodium bicarbonate, water, and brine, dried ($MgSO_4$), and concentrated in vacuo to give 9.7 g. of crude material as a white solid. Flash chromatography on Merck 9385 silica gel (970 g.) eluting with 3:1 hexanes:ethyl acetate

gave 6.87 g. of (S)-4-[[(3-acetylthio)-2-methyl-1-oxopropyl]amino]benzoic acid, methyl ester as a white solid; m.p. 140 -141°. TLC (silica gel; hexanes:ethyl acetate, 3:1) $R_f$ = 0.14.

| Anal. calc'd. for $C_{14}H_{17}NO_4S$: | | | | |
|---|---|---|---|---|
| | C, 56.93; | H, 5.80; | N, 4.74; | S, 10.86 |
| Found: | C, 56.96; | H, 5.81; | N, 4.74; | S, 10.98. |

b) (S)-4-[(3-Mercapto-2-methyl-1-oxopropyl)amino]benzoic acid

A solution of the methyl ester product from part (a) (6.85 g., 23.2 mmole) in methanol (160 ml.) was cooled in an ice bath under nitrogen and treated dropwise with 1N sodium hydroxide solution (69.6 ml., 69.6 mmole). After the addition was completed, the reaction mixture was stirred cold for 15 minutes and then allowed to warm to ambient temperature overnight. The reaction mixture was concentrated *in vacuo* to remove the methanol. The aqueous layer remaining was diluted with water (200 ml.) and washed with chloroform (2 x 70 ml.). The aqueous layer was acidified to pH 1 with concentrated HCl and extracted with 3 x 200 ml. of ethyl acetate. The combined organic extract was washed with 70 ml. of water and brine, dried ($MgSO_4$) and concentrated *in vacuo* to yield 5.15 g. of crude material. Flash chromatography in 2 batches on a column of Merck 9385 silica gel (300 g.) eluting with dichloromethane:methanol:acetic acid, 60:1:1 gave 2 g. of (S)-4-[(3-mercapto-2-methyl-1-oxopropyl)amino]benzoic acid as a white solid; m.p. 222 - 224°; $[\alpha]_D$ = -90.2° (c = 0.5, methanol). TLC (silica gel; benzene:acetic acid, 4:1) $R_f$ = 0.33 (minor impurity at 0.24).

| Anal. calc'd. for $C_{11}H_{13}NO_3S$: | | | | | |
|---|---|---|---|---|---|
| | C, 55.21; | H, 5.48; | N, 5.85; | S, 13.40; | SH, 13.82 |
| Found: | C, 54.99; | H, 5.56; | N, 5.75; | S, 13.01; | SH, 13.52. |

## Example 3

4-[[2-(Mercaptomethyl)-1-oxopentyl]amino]benzoic acid

a) 2-Propyl-3-acetylthiopropionic acid

A solution of potassium hydroxide pellets (11.22 g., 200 mmole) in absolute ethanol (120 ml.) was added to a stirred solution of diethyl propylmalonate (22.22 g., 100 mmole) in absolute ethanol (60 ml.) over a period of 30 minutes. After the mixture stood overnight at room temperature, the crystalline precipitate was filtered off. This material (10 g., 43 mmole) was dissolved in water (35 ml.) and a solution of potassium hydroxide pellets (2.6 g., 64 mmole) in water (50 ml.) was added and the reaction mixture was refluxed for 2 hours. The mixture was cooled to 5°, concentrated HCl (8.9 ml., 107 mmole) was added over 20 minutes while keeping the temperature below 10°, and the mixture was extracted with ether (3 x 75 ml.). The combined ether extracts were washed with saturated sodium chloride solution, dried ($MgSO_4$), and concentrated under reduced pressure to give 6.05 g. of n-propyl malonic acid, m.p. 94 - 95°.

n-Propyl malonic acid (3.0 g., 20 mmole) was dissolved in water (15 ml.) and neutralized by the slow addition of 25% aqueous dimethylamine. An additional amount of n-propyl malonic acid (3.0 g., 20 mmole) was added with stirring and the resulting solution was cooled to 0°. Aqueous formaldehyde (4.16 ml.of 33% solution) was added with cooling and stirring. After standing overnight the solution precipitated 3.42 g. of crystalline propyl (dimethylaminomethyl) malonic acid; m.p. 119 - 120°.

The propyl (dimethylaminomethyl) malonic acid (3.42 g., 17 mmole) was suspended in water (10 ml.) and the solution was neutralized by the addition of 10% aqueous sodium hydroxide. The resulting solution was refluxed overnight under a nitrogen atmosphere, then cooled and acidified with concentrated HCl (10 ml.). The mixture was extracted with ether (3 x 50 ml.), and the combined extracts were washed with water (1 x 20 ml.) and extracted with saturated sodium bicarbonate (3 x 30 ml.). The combined sodium bicarbonate extracts were acidified with concentrated HCl and extracted with ether (3 x 50 ml.). The ether extracts were washed with saturated sodium chloride (2 x 20 ml.), dried ($Na_2SO_4$), and concentrated under reduced pressure to give 1.55 g. of propylacrylic acid as an oily crude product.

The crude propylacrylic acid (1.55 g., 13 mmole) and thiolacetic acid (1.42 g., 18 mmole) with the addition of a few crystals of 2,2′-azobis[2-methylpropanenitrile] were refluxed for 4 hours and allowed to stand at room temperature for 20 hours. The reaction mixture was concentrated under reduced pressure, and residual thiolacetic acid was chased with toluene to give 1.51 g. of 2-propyl-3-acetylthiopropionic acid.

b) 4-[[2-[(Acetylthio)methyl]-1-oxopentyl]amino]benzoic acid, methyl ester

2-Propyl-3-acetylthiopropionic acid (1.91 g., 10 mmole) was dissolved in freshly distilled ether (20 ml.) and cooled to -5°. Oxalyl chloride (0.88 ml., 10 mmole) was added dropwise followed by N,N-dimethylformamide (3 drops). The ice bath was removed and the reaction was allowed to warm to room temperature as gas evolved. After stirring at room temperature for 3 hours, the reaction was a clear yellow solution with a small amount of a gummy yellow precipitate. The solvent was removed in vacuo and the residue was chased with tetrahydrofuran. The resulting yellow oil was dissolved in dichloromethane (10 ml.), cooled to 0°, and added to a solution of 4-aminobenzoic acid, methyl ester (1.52 g., 10 mmole) and diisopropylethylamine (1.75 ml., 10 mmole) in dry dichloromethane (30 ml.) at 0°. After stirring at 0° (10 minutes) and at room temperature (17 hours), the reaction was washed with saturated sodium bicarbonate, HCl (1 M), and saturated sodium chloride. The organic phase was dried ($MgSO_4$), filtered, and the solvent removed in vacuo to give 2.56 g. of the desired product as a yellow solid. Recrystallization from dichloromethane and hexane provides 2.2 g. of 4-[[2-[(acetylthio)methyl]-1-oxopentyl]amino]benzoic acid, methyl ester as white needles; m.p. 102.5 - 103.5°.

c) 4-[[2-(Mercaptomethyl)-1-oxopentyl]amino]benzoic acid

Sodium hydroxide (1M, 25 ml., 25 mmole) and methanol (25 ml.) were degassed with argon and added to 4-[[2-[(acetylthio)methyl]-1-oxopentyl]amino]benzoic acid, methyl ester (2.0 g., 6.18 mmole) under argon. Upon stirring for 10 minutes all of the starting material dissolved and the clear yellow reaction was allowed to stir for 16 hours at room temperature. The reaction mixture was washed with ether, acidified to pH 1 with concentrated HCl, and extracted with ethyl acetate. The organic extracts were combined, dried ($MgSO_4$), filtered, and the solvent removed. The white solid residue was purified by flash chromatography (150 g. of Whatman LPS-1; 5% acetic acid, 20% ethyl acetate, 75% hexane) to give 1.34 g. of 4-[[2-(mercaptomethyl)-1-oxopentyl]amino]benzoic acid as a white solid; m.p. 207.5 - 208.5°. TLC (silica gel; 5% acetic acid, 40% ethyl acetate, 55% hexane) $r_f$ = 0.39.

| Anal. calc'd. for $C_{13}H_{17}NO_3S$: | | | | | |
|---|---|---|---|---|---|
| | C, 58.40; | H, 6.41; | N, 5.24; | S, 11.99; | SH, 12.37 |
| Found: | C, 58.49; | H, 6.47; | N, 5.21; | S, 11.79; | SH, 12.32. |

Example 4

4-[[2-(Mercaptomethyl)-3-methyl-1-oxobutyl]amino]benzoic acid

a) 2-[(Acetylthio)methyl]-3-methylbutanoic acid

Potassium hydroxide (100 g., 1.79 mole) was dissolved with cooling in distilled water (100 ml.). Diethyl isopropyl malonate (100 ml., 488 mmole) was added and the two phase reaction mixture was heated at 70° for 18 hours. The homogeneous, clear brown reaction mixture was cooled to room temperature, acidified to a pH of 1 with concentrated HCl, and extracted with ethyl acetate. The organic extracts were dried ($MgSO_4$), filtered, and the solvent removed in vacuo to yield 80.79 g. of crude product. Recrystallization from isopropyl ether and hexane provides 67.5 g. of isopropyl malonic acid as a white solid.

Isopropyl malonic acid (65 g., 450 mmole) was dissolved in distilled water (400 ml.) and 37% aqueous formaldehyde (37.9 g., 470 mmole) and 40% aqueous dimethylamine (52.6 g., 470 mmole) were added and the mixture was stirred for 16 hours. The clear yellow solution was then heated at 80° for 12 hours during which time a gas evolved. After cooling to room temperature, the reaction was acidified to pH 1 with concentrated HCl, and extracted with ether and dichloromethane. The combined organic extracts were dried ($MgSO_4$), filtered and the solvent removed in vacuo to give 42.84 g. of isopropyl acrylic acid as a yellow oil.

Thiolacetic acid (22 ml., 307 mmole) and isopropyl acrylic acid (10 g., 87.6 mmole) were combined and heated at 80° for 70 minutes and then allowed to stir at room temperature overnight. The thiolacetic acid was removed by distillation in vacuo (aspirator, 40 - 45°) and the residue was chased several times with toluene to yield 20.04 g. of a clear yellow liquid. A portion (6.67 g.) of this material was purified by flash chromatography (300 g. of Whatman LPS-1, 15% acetone and hexane) to provide 3.896 g. of 2-[(acetylthio)-methyl]-3-methylbutanoic acid as a yellow oil.

b) 4-[[2-[(Acetylthio)methyl]-3-methyl-1-oxobutyl]amino]benzoic acid, methyl ester

The 2-[(acetylthio)methyl]-3-methylbutanoic acid (1.556 g., 8.18 mmole) was dissolved in freshly distilled ether (20 ml.) and cooled to -5°. Oxalyl chloride (0.71 ml., 8.18 mmole) was added dropwise followed by N,N-dimethylformamide (3 drops). The ice bath was removed and the reaction was allowed to warm to room temperature as gas evolved. After stirring at room temperature for 1.66 hours, the reaction was a clear yellow solution with a small amount of gummy yellow precipitate. The solvent was removed in vacuo and the residue was chased with tetrahydrofuran. The resulting yellow oil was dissolved in dichloromethane (10 ml.), cooled to 0°, and added to a solution of 4-aminobenzoic acid, methyl ester (1.24 g. 8.18 mmole) and diisopropylethyl amine (1.42 ml., 8.18 mmole) in dry dichloromethane (30 ml.) at 0°. After stirring 10 minutes at 0° and then 16 hours at room temperature, the reaction was washed with saturated sodium bicarbonate, 1.0 M HCl, and saturated sodium chloride. The organic phase was dried ($MgSO_4$), filtered, and the solvent removed in vacuo to give 2.22 g. of 4-[[2-[(acetylthio)methyl]-3-methyl-1-oxobutyl]amino]benzoic acid, methyl ester as a yellow solid.

c) 4-[[2-(Mercaptomethyl)-3-methyl-1-oxobutyl]amino]benzoic acid

Sodium hydroxide (1.0 M, 25 ml., 25 mmole) and methanol (25 ml.) were degassed with argon and added to 4-[[2-[(acetylthio)methyl]-3-methyl-1-oxobutyl]amino]benzoic acid, methyl ester (2.0 g., 6.18 mmole) under argon. Upon stirring for 10 minutes all of the starting material dissolved and the clear yellow reaction was allowed to stir for 5 hours at room temperature. The reaction mixture was then acidified to pH of 1 with concentrated HCl and extracted with ethyl acetate. The organic extracts were combined, dried ($MgSO_4$), filtered, and then the solvent removed. The white solid residue was purified by flash chromatography (160 g. of Whatman LPS-1; 5% acetic acid, 20% ethyl acetate, 75% hexane) to yield 238 mg. of 4-[[2-(mercaptomethyl)-3-methyl-1-oxobutyl]amino]benzoic acid as a white solid; m.p. 250 - 270° (dec.). TLC (silica gel; 5% acetic acid, 40% ethyl acetate, 55% hexane) $R_f$ = 0.34.

| Anal. calc'd. for $C_{13}H_{17}NO_3S$: | | | | | |
|---|---|---|---|---|---|
| | C, 58.40; | H, 6.41; | N, 5.24; | S, 11.99; | SH, 12.37 |
| Found: | C, 58.41; | H, 6.45; | N, 5.05; | S, 11.78; | SH, 12.27. |

Example 5

4-[[2-(Mercaptomethyl)-4-methyl-1-oxopentyl]amino]benzoic acid

a) 4-[[2-[(Acetylthio)methyl]-4-methyl-1-oxopentyl]amino]benzoic acid, methyl ester

2-[(Acetylthio)methyl]-4-methylpentanoic acid (1.5 g., 7.34 mmole) [prepared as described by Sundeen et al. in U.S. Patent 4,235,885 in Example 1] was dissolved in freshly distilled ether (15 ml.) and cooled to -5°. Oxalyl chloride (0.64 ml., 7.34 mmole) was added dropwise followed by N,N-dimethylformamide (3 drops). The ice bath was removed and the reaction was allowed to warm to room temperature as gas evolved. After stirring at room temperature for one hour, the reaction was a clear yellow solution with a small amount of gummy precipitate. The solvent was removed in vacuo and the residue was chased with tetrahydrofuran. The resulting yellow oil was dissolved in dichloromethane (10 ml.), cooled to 0°, and added to a solution of 4-aminobenzoic acid, methyl ester (1.11 g., 7.34 mmole) and diisopropylethylamine (1.42 ml., 8.18 mmole) in dry dichloromethane (20 ml.) at 0°. After stirring for 10 minutes at 0° and then for one hour at room temperature, the reaction was washed with saturated sodium bicarbonate, 1.0 M HCl, and saturated sodium chloride. The organic phase was dried ($MgSO_4$), filtered, and the solvent was removed in vacuo to yield 2.16 g. of crude product as a yellow solid. Purification by flash chromatography (130 g. of Whatman LPS-1, 20% ethyl acetate, hexane) followed by a second chromatography (100 g. of Whatman LPS-1, 18% ethyl acetate, hexane) gave 1.89 g. of 4-[[2-[(acetylthio)methyl]-4-methyl-1-oxopentyl]amino]-benzoic acid, methyl ester. TLC (silica gel; 20% ethyl acetate, hexane) $R_f$ = 0.20.

b) 4-[[2-(Mercaptomethyl)-4-methyl-1-oxopentyl]amino]benzoic acid

The methyl ester product from part (a) (1.89 g., 5.33 mmole) was dissolved in methanol (35 ml.) and degassed by bubbling argon through the mixture. Sodium hydroxide (1.0 M, 16 ml. 16 mmole) was added and the reaction was allowed to stir for 2 hours at room temperature. The reaction mixture was concentrated in vacuo; and the residue was dissolved in water (200 ml.), and washed with dichloromethane. The aqueous layer was acidified to a pH of 1 with concentrated HCl and extracted with ethyl acetate. The organic extracts were combined, dried ($MgSO_4$), filtered, and the solvent removed. The white residue was purified by flash chromatography (130 g. of Whatman LPS-1, 5% acetic acid, 10% ethyl acetate, 85% hexane) to give 1.25 g. of 4-[[2-(mercaptomethyl)-4-methyl-1-oxopentyl]amino]benzoic acid as a white solid; m.p. 200 - 201°. TLC (silica gel; 35% ethyl acetate, 5% acetic acid, 60% hexane) $R_f$ = 0.66.

| Anal. calc'd. for $C_{14}H_{19}NO_3S$: | | | | | |
|---|---|---|---|---|---|
| | C, 59.76; | H, 6.81; | N, 4.98; | S, 11.39; | SH, 11.75 |
| Found: | C, 59.66; | H, 6.96; | N, 4.90, | S, 11.45; | SH, 11.79. |

Example 6

4-[[2-(Mercaptomethyl)-1-oxobutyl]amino]benzoic acid

a) 2-[(Acetylthio)methyl]butanoic acid

Diethylethyl malonate (20 g., 106.3 mmole) was placed in a flask with potassium hydroxide (22.9 g., 408.1 mmole) and water (18.3 ml.). The reaction was stirred at reflux for 27 hours then cooled to room temperature. The reaction was diluted with water (200 ml.), washed with ethyl acetate, acidified to pH 1 with

concentrated HCl, and extracted with ethyl acetate. The organic phase was dried ($MgSO_4$), filtered, and concentrated in vacuo to yield a yellow solid which was recrystallized from isopropyl ether and hexane to yield 46.7 g. of ethylmalonic acid as a yellow crystalline solid; m.p. 112°.

The ethylmalonic acid (45 g., 340 mmole) was dissolved in water (300 ml.), and 37% aqueous formaldehyde (29 g., 358 mmole) and dimethylamine (49 g., 358 mmole) were added. The solution was stirred at room temperature for 24 hours and then the reaction was heated to 80° until the solid dissolved and the evolution of gas ceased. After 5 hours, the reaction was acidified to pH 1 with concentrated HCl, extracted with dichloromethane, dried ($MgSO_4$), filtered, and concentrated in vacuo (bath temperature of 20°) to give 16.2 g. of ethyl acrylic acid as a clear colorless liquid.

A mixture of the ethyl acrylic acid (16 g., 159.8 mmole) and thiolacetic acid (40 ml., 559.3 mmole) was stirred for one hour at room temperature and then at 80° for 2 hours. The reaction was cooled to room temperature and the thiolacetic acid was azeotroped with toluene in vacuo (aspirator) to give 25 g. of a yellow oil. Purification of 5 g. of this material by flash chromatography (Whatman LPS-1; 15% acetone, hexane) yields 4.46 g. of 2-[(acetylthio)methyl]butanoic acid.

b) 4-[[2-[(Acetylthio)methyl]-1-oxobutyl]amino]benzoic acid, methyl ester

A solution of 2-[(acetylthio)methyl]butanoic acid (1.87 g., 10.61 mmole) in freshly distilled ether (15 ml.), under argon, was cooled to 0°. Oxalyl chloride (0.93 ml., 10.61 mmole) and N,N-dimethylformamide (catalytic amount) were added and the yellow solution was stirred at room temperature. After 2 hours, the reaction was concentrated in vacuo and chased with tetrahydrofuran. The resulting yellow oil was dissolved in dichloromethane (10 ml.), cooled to 0°, and added to a solution of 4-aminobenzoic acid, methyl ester (1.6 g., 10.61 mmole) and diisopropylethylamine (1.85 ml., 10.61 mmole) in dichloromethane (20 ml.) at 0°. The orange solution was warmed to room temperature and stirred under argon for 2 hours. The reaction mixture was then washed with saturated sodium bicarbonate, 1.0 M HCl, and saturated sodium chloride. The organic phase was dried ($MgSO_4$), filtered, and concentrated in vacuo to yield an orange solid. Purification by flash chromatography (140 g. of Whatman LPS-1 silica gel; 23% ethyl acetate, hexane) yielded a white solid; m.p. 109 -111°. Further purification by recrystallization from dichloromethane and hexane yielded 2.22 g. of 4-[[2-[(acetylthio)methyl]-1-oxobutyl]amino]benzoic acid, methyl ester as a white solid.

| Anal. calc'd. for $C_{15}H_{19}NO_4S$: | | | | |
|---|---|---|---|---|
| | C, 58.23; | H, 6.19; | N, 4.53; | S, 10.36 |
| Found: | C, 58.32; | H, 6.21; | N, 4.40; | S, 10.05. |

c) 4-[[2-(Mercaptomethyl)-1-oxobutyl]amino]benzoic acid

The methyl ester product from part (b) (1.73 g., 56.59 mmole) was dissolved in methanol (37 ml.), degassed with argon, and cooled to 0°. Over a ten minute period, 1.0 N sodium hydroxide (16.8 ml., 16.8 mmole) was added, and the reaction was again degassed with argon and stirred for 2 hours under argon at room temperature. The reaction was then concentrated in vacuo, diluted with water (50 ml.), acidified to pH 1 with concentrated HCl, and extracted with ethyl acetate. The organic phase was dried ($MgSO_4$), filtered, and concentrated in vacuo to yield 1.33 g. of a white solid. Purification by flash chromatography (85 g. of Whatman LPS-1; 12% ethyl acetate, 5% acetic acid, 83% hexane) yielded the product eluting with the starting material. This material was redissolved in 1.0 N sodium hydroxide and extracted with chloroform. The aqueous phase was acidified to pH 1, extracted with ethyl acetate, dried ($MgSO_4$), and concentrated in vacuo to a white solid. Purification by flash chromatography (146 g. of Whatman LPS-1; 10% ethyl acetate, 5% acetic acid, 85% hexane) yielded 1.24 g. of 4-[[2-(mercaptomethyl)-1-oxobutyl]amino]benzoic acid as a white solid; m.p. 216 - 217° (dec.). TLC (silica gel; 35% ethyl acetate, 5% acetic acid, 60% hexane) $R_f$ = 0.61.

| Anal. calc'd. for $C_{12}H_{15}NO_3S$: | | | | | |
|---|---|---|---|---|---|
| | C, 56.67; | H, 5.99; | N, 5.51; | S, 12.61; | SH, 13.00 |
| Found: | C, 56.65; | H, 6.15; | N, 5.30; | S, 12.57; | SH, 13.26. |

## Example 7

### 3-[[2-(Mercaptomethyl)-1-oxo-3-phenylpropyl]amino]benzoic acid

#### a) 3-[[2-[(Acetylthio)methyl]-1-oxo-3-phenylpropyl]amino]benzoic acid, methyl ester

3-Acetylthio-2-benzylpropanoic acid (2.0 g., 8.84 mmole) was dissolved in freshly distilled ether (20 ml.) and cooled to -5°. Oxalyl chloride (0.77 ml., 8.84 mmole) was added dropwise followed by N,N-dimethylformamide (3 drops). The ice bath was removed and the reaction was allowed to warm to room temperature as gas evolved. After stirring at room temperature for 1.5 hours, the reaction was a clear yellow solution with a small amount of gummy yellow precipitate. The solvent was removed in vacuo and the residue was chased with tetrahydrofuran. The resulting yellow-green oil was dissolved in freshly distilled dichloromethane (10 ml.), cooled to 0°, and added to a solution of 3-aminobenzoic acid, methyl ester (1.13 g., 8.84 mmole) and diisopropylethylamine (1.54 ml., 8.84 mmole) in dry dichloromethane (20 ml.) at 0°. After stirring for ten minutes at 0° and then overnight at room temperature, the reaction was washed with saturated sodium bicarbonate, 1.0 M HCl, and saturated sodium chloride. The organic phase was dried ($MgSO_4$), filtered, and the solvent was removed in vacuo to yield 2.77 g. of crude product as a brown oil. Purification by flash chromatography (120 g. of Whatman LPS-1; 20% ethyl acetate, hexane) yielded 1.76 g. of 3-[[2-[(acetylthio)methyl]-1-oxo-3- phenylpropyl]amino]benzoic acid, methyl ester as a tan solid; m.p. 101 - 102.5°. TLC (silica gel; 20% ethyl acetate, hexane) $R_f$ = 0.13.

#### b) 3-[[2-(Mercaptomethyl)-1-oxo-3-phenylpropyl]amino]benzoic acid

The methyl ester product from part (a) (1.53 g., 4.11 mmole) was dissolved in 12 ml. of methanol, sodium hydroxide (1.0 M, 12 ml., 12 mmole) was added, and the reaction was degassed in vacuo and placed under argon. After stirring for 2.5 hours at room temperature, an additional amount of sodium hydroxide (8 ml.) was added. After stirring for an additional 2 hours, the reaction was acidified to pH 1 with concentrated hydrochloric acid. The methanol was removed in vacuo and the aqueous residue was extracted with ethyl acetate. The organic extracts were combined, dried ($MgSO_4$), filtered, and the solvent removed. The residue was purified by flash chromatography (100 g. of Whatman LPS-1, 5% acetic acid, 20% ethyl acetate, 75% hexane) to yield 1.1 g. of 3-[[2-(mercaptomethyl)-1-oxo-3-phenylpropyl]amino]-benzoic acid as a white solid; m.p. 173 - 175°. TLC (silica gel; 5% acetic acid, 40% ethyl acetate, 55% hexane) $R_f$ = 0.33.

Anal. calc'd. for $C_{17}H_{17}NO_3S$:
C, 64.74; H, 5.43; N, 4.44; S, 10.17; SH, 10.49
Found: C, 64.89; H, 5.42; N, 4.37; S, 10.18; SH, 10.67.

## Example 8

### (S)-3-[(3-Mercapto-2-methyl-1-oxopropyl)amino]benzoic acid

a) (S)-3-[[3-[(Acetylthio)methyl]-2-methyl-1-oxopropyl]amino]benzoic acid, methyl ester

A mixture of 3-aminobenzoic acid, methyl ester (1.01 g., 6.7 mmole) and diisopropylethylamine (1.3 ml., 7.4 mmole) was dissolved in freshly distilled dichloromethane (20 ml.). After cooling to 0°, D-3-(acetylthio)-2-methylpropanoyl chloride (1.0 ml., 6.7 mmole) was added dropwise and the reaction was allowed to stir and warm to room temperature over 15 hours. The reaction mixture was then washed with saturated sodium bicarbonate, 1.0 M HCl, and saturated sodium chloride solutions. The organic phase was then dried ($MgSO_4$), filtered, and the solvent removed to give 2.12 g. of crude product as a brown oil. Purification by flash chromatography (100 g. of Whatman LPS-1; 20% ethyl acetate, hexane) yielded 1.62 g. of (S)-3-[[3-[(acetylthio)methyl]-2-methyl-1-oxopropyl]amino]benzoic acid, methyl ester as a white, glassy solid. TLC (silica gel; 20% ethyl acetate, hexane) $R_f$ = 0.09.

b) (S)-3-[(3-Mercapto-2-methyl-1-oxopropyl)amino]benzoic acid

The methyl ester product from part (a) (1.62 g., 5.49 mmole) was dissolved in methanol (22 ml.), sodium hydroxide (1.0 M, 22 ml., 22 mmole) was added and the reaction was degassed in vacuo and placed under argon. After stirring for 2 hours at room temperature, the reaction was washed with ethyl acetate. The aqueous layer was acidified to pH 1 with concentrated HCl and extracted with ethyl acetate. The organic extracts were combined, dried ($MgSO_4$), filtered, and the solvent removed. The residue was purified by flash chromatography (110 g. of Whatman LPS-1; 5% acetic acid, 20% ethyl acetate, 75% hexane) to yield 968 mg. of (S)-3-[(3-mercapto-2-methyl-1-oxopropyl)amino]benzoic acid as a white solid; m.p. 195 - 196°; $[\alpha]_D$ = -73.6° (c = 0.72, methanol). TLC (silica gel; 5% acetic acid, 40% ethyl acetate, 55% hexane) $R_f$ = 0.31.

| Anal. calc'd. for $C_{11}H_{13}NO_3S$: | | | | | |
|---|---|---|---|---|---|
| | C, 55.21; | H, 5.48; | N, 5.85; | S, 13.40; | SH, 13.82 |
| Found: | C, 55.22; | H, 5.37; | N, 5.89; | S, 13.13; | SH, 13.99. |

## Example 9

### 3-[[2-(Mercaptomethyl)-4-methyl-1-oxopentyl]amino]benzoic acid

a) 3-Aminobenzoic acid, methyl ester, hydrochloride

A suspension of 3-aminobenzoic acid (10 g., 72.9 mmole) in methanol (200 ml.) was cooled to -10° and treated dropwise with thionyl chloride (17.3 g. 146 mmole) keeping the temperature below -5°. After the addition was complete, the reaction mixture was allowed to warm to ambient temperature overnight. The mixture was concentrated in vacuo, and the residue was twice triturated with ether to give 13.2 g. of 3-aminobenzoic acid, methyl ester, hydrochloride as a white solid; m.p. 207 -216°. TLC (silica gel; chloroform:methanol:acetic acid, 18:1:1) $R_f$ = 0.70.

b) 3-[[2-[(Acetylthio)methyl]-4-methyl-1-oxopentyl]amino]benzoic acid, methyl ester

A solution of 2-[(acetylthio)methyl]-4-methylpentanoic acid (2.85 g., 13.95 mmole) in dry ether (30 ml.) under nitrogen was treated with oxalyl chloride (1.77 g., 13.95 mmole) followed by N,N-dimethylformamide (3 drops). The reaction mixture was stirred at room temperature for 2 hours, concentrated in vacuo, and then twice concentrated from dry tetrahydrofuran (30 ml.) to give 2-[(acetylthio)methyl]-4-methylpentanoyl chloride.

A solution of 3-aminobenzoic acid, methyl ester, hydrochloride (2.62 g., 13.95 mmole) in dry dichloromethane (20 ml.) was treated with diisopropylethylamine (1.8 g., 13.95 mmole), and the reaction mixture was cooled to -5° under nitrogen. This mixture was treated concurrently with a solution of the above 2-[-(acetylthio)methyl]-4-methylpentanoyl chloride in dichloromethane (20 ml.) and with diisopropylethylamine (1.8 g., 13.95 mmole). The mixture was stirred cold for 2 hours and then allowed to come to ambient temperature overnight. The reaction mixture was concentrated in vacuo. The residue was dissolved in ethyl acetate (100 ml.), and this solution was washed with 25 ml. portions of 10% potassium bisulfate, water, 5% sodium bicarbonate, water, and brine, dried ($MgSO_4$), and concentrated in vacuo to give 4.6 g. of crude product. Flash chromatography (400 g. of Merck 9385 silica gel, eluting with 7:1 petroleum ether:acetone) gives 3.8 g. of 3-[[2-[(acetylthio)methyl]-4-methyl-1-oxopentyl] amino]benzoic acid, methyl ester as an oil. TLC (silica gel; petroleum ether:acetone, 5:1) $R_f$ = 0.23.

c) 3-[[2-(Mercaptomethyl)-4-methyl-1-oxopentyl]amino]benzoic acid

A solution of the methyl ester product from part (b) (3.7 g., 10.9 mmole) in methanol (80 ml.) was cooled in an ice bath under nitrogen and treated dropwise with 1N sodium hydroxide (32.9 ml., 32.9 mmole). The reaction mixture was stirred cold for 15 minutes and then allowed to warm to ambient temperature overnight. The methanol was removed in vacuo, and the aqueous residue was diluted with water (100 ml.). The mixture was acidified with concentrated HCl and extracted with 3 x 75 ml. of ethyl acetate. The organic extract was washed with water and brine, dried ($MgSO_4$), and concentrated in vacuo to give 2.9 g. of crude product. Flash chromatography [300 g. of Merck 9385 silica gel; eluting with toluene:acetic acid (10:1)] gave 2.02 g. of 3-[[2-(mercaptomethyl)-4-methyl-1-oxopentyl]amino]benzoic acid as a white solid; m.p. 206 - 210°.

| Anal. calc'd. for $C_{14}H_{19}NO_3S$: | | | | | |
|---|---|---|---|---|---|
| | C, 59.76; | H, 6.81; | N, 4.98; | S, 11.39; | SH, 11.75 |
| Found: | C, 60.06; | H, 6.78; | N, 4.94; | S, 11.17; | SH, 11.45. |

## Example 10

### 4-[[3,3,3-Trifluoro-2-(mercaptomethyl)-1-oxopropyl]amino]benzoic acid

a) 2-[(Acetylthio)methyl]-3,3,3-trifluoropropanoyl chloride

α-Trifluoromethyl acrylic acid (10 g., 71 mmole) [prepared as described in J. Chem Soc., 1954, p. 371] was cooled in a salt-ice water bath, stirred, and treated portionwise with 97% thiolacetic acid (5.7 ml., 75 mmole). After the addition, the yellow liquid was stored in the cold for one hour, allowed to warm to room temperature, and distilled to yield 14 g. of 2-[(acetylthio)methyl]-3,3,3-trifluoropropanoic acid as a light yellow oil; b.p. 149 - 153° (13 mm.).

This acid (7.0 g., 32 mmole) was treated with redistilled thionyl chloride (18 ml., 25 mmole) and the mixture was refluxed for 3 hours. After removing excess thionyl chloride on a rotary evaporator, the residue was distilled to give 2-[(acetylthio)methyl]-3,3,3-trifluoropropanoyl chloride as a pale yellow oil; b.p. 80 - 82° (16 mm.).

b) 4-[[3,3,3-Trifluoro-2-[(acetylthio)methyl]-1-oxopropyl]amino]benzoic acid

4-Aminobenzoic acid (685 mg., 5 mmole) was suspended in acetonitrile (10 ml.). Bis(trimethylsilyl)-trifluoroacetamide (4 ml.) was added and the solid went into solution immediately. The resulting solution was stirred for 3 hours at room temperature. The 2-[(acetylthio)methyl]-3,3,3-trifluoropropanoyl chloride (1 g.,4.26 mmole) in acetonitrile (2 ml.) was added, and the mixture was stirred overnight at room temperature. The reaction mixture was concentrated on a rotary evaporator. Water (20 ml.) was added to the residue and this mixture was stirred for 15 minutes. It was then partitioned between ethyl acetate and 5% potassium bisulfate. The ethyl acetate solution was washed with brine and concentrated to a yellow solid. This was chromatographed on Merck silica (300 ml.) eluting with dichloromethane:methanol:acetic acid, 40:1:1 to give 810 mg. of 4-[[3,3,3-trifluoro-2-[(acetylthio)methyl]-1-oxopropyl]amino]benzoic acid as a white solid. TLC (silica gel; dichloromethane:methanol:acetic acid, 40:1:1) $R_f$ = 0.72.

c) 4-[[3,3,3-Trifluoro-2-(mercaptomethyl)-1-oxopropyl]amino]benzoic acid

The product from part (b) (810 mg., 2.42 mmole) was stirred at 0° under argon with concentrated ammonium hydroxide (1.6 ml.) and water (3.5 ml.) for 15 minutes. 5% Potassium bisulfate (100 ml.) was added and the resulting solution was extracted with ethyl acetate. The combined ethyl acetate layers were washed with brine, dried ($MgSO_4$), and concentrated to a beige solid which was then chromatographed on Whatman LPS-1 silica (300 ml.) using dichloromethane:methanol:acetic acid (40:1:1) as the eluant to give 270 mg. of 4-[[3,3,3-trifluoro-2-(mercaptomethyl)-1-oxopropyl]amino]benzoic acid as a white solid; m.p. 217°; $[\alpha]_D$ = +2.2° (c = 0.23, methanol). TLC(silica gel; dichloromethane: methanol:acetic acid, 20:1:1) $R_f$ = 0.50.

| Anal. calc'd. for $C_{11}H_{10}F_3NO_3S$ | | | | | | |
|---|---|---|---|---|---|---|
| | C, 45.05; | H, 3.44; | N, 4.78; | S, 10.93; | SH, 11.28; | F, 19.44 |
| Found: | C, 45.41: | H, 3.39; | N, 4.64; | S, 11.08; | SH, 11.62; | F, 19.66. |

## Example 11

3-[[3,3,3-Trifluoro-2-(mercaptomethyl)-1-oxopropyl]amino]benzoic acid

a) 3-[[3,3,3-Trifluoro-2-[(acetylthio)methyl]-1-oxopropyl]amino]benzoic acid

3-Aminobenzoic acid (2.0 g., 14.58 mmole) was placed in a flask under argon and suspended in dry acetonitrile (15 ml.). The suspension was cooled to 0° and bis(trimethylsilyl)trifluoroacetamide (7.7 ml., 29.2 mmole) was added. Within 20 minutes all of the solid had dissolved and the 2-[(acetylthio)methyl]-3,3,3-trifluoropropanoyl chloride (1.49 g., 6.35 mmole) was added dropwise. The reaction was allowed to stir and warm to room temperature overnight. The reaction mixture was partitioned between ethyl acetate and 1.0 M HCl and the aqueous layer was extracted with ethyl acetate. The combined organic extracts were dried ($MgSO_4$), filtered, and the solvent removed to yield 2.0 g. of crude product as a white solid. The residue was purified by flash chromatography (100 g. of Whatman LPS-1, 5% acetic acid, 20% ethyl acetate, and 75% hexane) to yield 1.54 g. of 3-[[3,3,3-trifluoro-2-[(acetylthio)methyl]-1-oxopropyl]amino]benzoic acid as a white solid; m.p. 205.5 - 206.5°.

b) 3-[[3,3,3-Trifluoro-2-(mercaptomethyl)-1-oxopropyl]amino]benzoic acid

The product from part (a) (500 mg., 1.49 mmole) was placed in a flask under argon and aqueous ammonium hydroxide (1.2 ml., 4.0 M) was added. The solid dissolved and the reaction became clear

yellow. After stirring for 40 minutes, the reaction was acidified with 1.0 M HCl and then extracted with ethyl acetate. The combined organic extracts were dried ($MgSO_4$) and the solvent was removed to yield 381 mg. of crude product as a yellow solid. Purification by preparative HPLC (YMC S345 50 x 200 mm column, 50 - 74% methanol in water containing 0.1% trifluoroacetic acid linear gradient over 50 minutes, 18.6 ml./min. flow rate, UV detection at 220 nm) gave 140 mg. of 3-[[3,3,3-trifluoro-2-(mercaptomethyl)-1-oxopropyl]-amino]benzoic acid as a white solid; m.p. 234 - 236° (dec.). TLC(silica gel; 5% acetic acid, 40% ethyl acetate, 55% hexane) $R_f$ = 0.33.

| Anal. calc'd. for $C_{11}H_{10}F_3NO_3S$: | | | | | | |
|---|---|---|---|---|---|---|
| | C, 45,05; | H, 3.44; | N, 4.78; | F, 19.44; | S, 10.93; | SH, 11.28 |
| Found: | C, 44.87; | H, 3.38; | N, 4.67; | F, 19.05; | S, 10.73; | SH, 11.58. |

## Example 12

### 4-[(3-Mercapto-1-oxo-2-phenylpropyl)amino]benzoic acid

#### a) 3-(Acetylthio)-2-phenylpropanoic acid

Thiolacetic acid (5.32 g., 70 mmole) was added dropwise to a solution of atropic acid (7.4 g., 50 mmole) in chloroform (75 ml.). The solution was allowed to stand overnight at room temperature. The solvent was removed on the rotary evaporator and the semi-solid residue was triturated with hexane to 9.3 g. of product; m.p. 96 - 98°. Recrystallization from cyclohexane gave 3-(acetylthio)-2-phenylpropanoic acid as a solid; m.p. 94 - 96°.

| Anal. calc'd. for $C_{11}H_{12}O_3S$: | | | |
|---|---|---|---|
| | C, 58.91; | H, 5.40; | S, 14.30 |
| Found: | C, 58.69; | H, 5.40; | S, 14.20. |

#### b) 4-[[3-(Acetylthio)-1-oxo-2-phenylpropyl]amino]benzoic acid, methyl ester

A solution of 3-(acetylthio)-2-phenylpropanoic acid (2.55 g., 11.37 mmole) in distilled ether (15 ml.) under argon was cooled to 0°. Oxalyl chloride (0.99 ml., 11.37 mmole) and N,N-dimethylformamide (3 drops) were added and the yellow solution was stirred at room temperature. After 2 hours, the reaction was concentrated *in vacuo* and chased with tetrahydrofuran. The resulting yellow oil was dissolved in dichloromethane (10 ml.), cooled to 0°, and added to a solution of 4-aminobenzoic acid, methyl ester (1.72 g., 11.37 mmole) and diisopropylethylamine (1.98 ml., 11.37 mmole) in dichloromethane (15 ml.) at 0°. The orange solution was warmed to room temperature and stirred under argon for 1 hour. The reaction mixture was washed with saturated sodium bicarbonate, 1N HCl, and saturated sodium chloride. The organic phase was dried ($MgSO_4$), filtered, and concentrated *in vacuo* to yield an orange foam. Purification by flash chromatography (445 g. of Whatman LPS-1; 23% ethyl acetate, hexane) yielded 3.66 g. of 4-[[3-(acetylthio)-1-oxo-2-phenylpropyl]amino]benzoic acid, methyl ester as a white solid. TLC (silica gel; 30% ethyl acetate, hexane) $R_f$ = 0.30.

#### c) 4-[(3-Mercapto-1-oxo-2-phenylpropyl)amino]benzoic acid

The methyl ester product from part (b) (2.94 g., 8.56 mmole) was dissolved in methanol (57 ml.), degassed with argon, and cooled to 0°. Over a ten minute period 1.0 N sodium hydroxide (26 ml., 26 mmole) was added. the reaction was again degassed with argon and stirred at room temperature under argon. Additional sodium hydroxide was added after one hour (17 ml., 17 mmole), and two hours (17 ml., 17 mmole), then the reaction was stirred for 45 minutes. The reaction was concentrated in vacuo, dissolved in water, acidified to pH 1 with concentrated HCl, and extracted with ethyl acetate. The organic phase was dried ($MgSO_4$), filtered, and concentrated in vacuo to yield 2.39 g. of a white solid. Purification three times by flash chromatography (160 g. of Whatman LPS-1; 15% ethyl acetate, 5% acetic acid, 80% hexane) yielded 330 mg. of 4-[(3-mercapto-1-oxo-2-phenylpropyl)amino]benzoic acid as an off white solid; m.p. 240 - 242° TLC (silica gel; ethyl acetate:acetic acid:hexane, 50:5:45) $R_f$ = 0.58.

| Anal. calc'd. for $C_{16}H_{15}NO_3S$: | | | | | |
|---|---|---|---|---|---|
| | C, 63.77; | H, 5.02; | N, 4.65; | S, 10.64; | SH, 10.97 |
| Found: | C, 64.05; | H, 5.35; | N, 4.68; | S, 10.40; | SH, 11.07. |

## Example 13

### 4-[[2-(Mercaptomethyl)-1-oxo-4-phenylbutyl]amino]benzoic acid

#### a) 2-[(Acetylthio)methyl]-4-phenylbutanoic acid

Phenethyl bromide (30 g., 160 mmole) was added, all at once, to a solution of sodium metal (6.5 g., 280 mmole) and diethyl malonate (45 g., 280 mmole) in absolute ethanol (90 ml.). After stirring for 30 minutes at room temperature, a white precipitate formed and the reaction temperature rose to 40°. Stirring was continued overnight, then the mixture was heated at reflux for 6 hours. The solvent was removed in vacuo and the residue was partitioned between water (250 ml.) and ether (3 x 200 ml.). The organic extracts were combined, washed with brine, and dried ($MgSO_4$). Removal of the ether in vacuo yielded an amber colored, viscous residue (46.2 g.). This material was distilled and the fraction boiling at 161 - 163° 5 mm Hg. was collected to give 19.5 g. of diethyl(phenylethyl)malonate.

Diethyl(phenylethyl) malonate (114.6 g., 430 mmole) was treated with 10% aqueous sodium hydroxide (600 ml.) and heated under reflux with stirring for 5 hours, cooled, and allowed to stand overnight. The reaction was then refluxed for an additional 3 hours. The resulting clear solution was made strongly acidic with 20% aqueous HCl and the white precipitate that formed was filtered to yield 82.6 g. of (phenylethyl) malonic acid as a white solid; m.p. 128 - 130° (dec.).

A suspension of the (phenylethyl) malonic acid (78.8 g., 380 mmole) in water (860 ml.) was treated with 40% aqueous dimethylamine (410 mmole) and formalin (33.3 g., 410 mmole). A clear solution formed promptly, and the mixture was allowed to stand overnight at room temperature. The resulting white precipitate was filtered, the filtrate was acidified with 10% potassium bisulfate, and the solid that formed was filtered and combined with the first crop to give 86.4 g. of phenylethyl dimethylaminomethyl malonic acid; m.p. 120 - 122° (dec.).

This malonic acid (86.4 g., 330 mmole) was mixed with water (3 l.) and heated under reflux for 1.5 hours during which carbon dioxide evolved and a clear solution was formed. The solution was cooled, acidified with potassium bisulfate, and the resulting white precipitate was filtered and dried to give 48.5 g. of (phenylethyl) acrylic acid as a white solid; m.p. 51 - 52° (dec.).

A mixture of the (phenylethyl) acrylic acid (17.6 g.) and thiolacetic acid (50 ml.) was stirred for 24 hours at room temperature during which time a clear solution formed. The solution was allowed to stand at room temperature for an additional week and then the excess thiolacetic acid was removed by water aspiration followed by a hot water bath and vacuum pump. The solid yellow residue was heated on a steam cone under high vacuum and then triturated in hexane to give 18.2 g. of white crystalline 2-[(acetylthio)methyl]-4-phenylbutanoic acid; m.p. 52 - 53°.

b) 4-[[2-[(Acetylthio)methyl]-1-oxo-4-phenylbutyl]amino]benzoic acid, methyl ester

A solution of 2-[(acetylthio)methyl]-4-phenylbutanoic acid (2.72 g., 10.8 mmole) in dry ether (25 ml.) under nitrogen was treated with oxalyl chloride (1.37 g., 10.8 mmole) followed by N,N-dimethylformamide (2 drops), and the mixture was stirred at ambient temperature. After one hour, the reaction mixture was concentrated in vacuo, and was then twice dissolved in toluene and concentrated in vacuo.

A solution of 4-aminobenzoic acid, methyl ester (1.63 g., 10.8 mmole) in dry dichloromethane (15 ml.) was cooled to -5° under nitrogen. The solution was treated concurrently with a solution of the above acid chloride in dry dichloromethane (15 ml.) and distilled diisopropylethylamine (1.39 g., 10.9 mmole). The reaction mixture was stirred cold for 2 hours, and then was allowed to warm to ambient temperature overnight. The mixture was concentrated in vacuo. The residue was dissolved in ethyl acetate (75 ml.) and washed with 25 ml. portions of 10% potassium bisulfate, water, 5% sodium bicarbonate, water, and brine, dried ($MgSO_4$), and concentrated in vacuo to give 4.3 g. of crude product. Flash chromatography on 400 g. of Merck 9385 silica gel, eluting with 6:1 petroleum ether:acetone gave 3.6 g. of partially purified product. A second flash chromatography on 350 g. of Merck 9385 silica gel, eluting with 10:1 toluene:acetone gave 3.25 g. of 4-[[2-[(acetylthio)methyl]-1-oxo-4-phenylbutyl]amino[benzoic acid, methyl ester as as white solid; m.p. 88 - 93°. TLC (silica gel; toluene:acetone, 10:1) $R_f$ = 0.53.

c) 4-[[2-(Mercaptomethyl)-1-oxo-4-phenylbutyl]amino]benzoic acid

A solution of the methyl ester product from part (b) (3.2 g., 8.3 mmole) in methanol (65 ml.) was cooled in an ice bath under nitrogen and treated dropwise with 1N sodium hydroxide solution (24.9 ml., 24.9 mmole). The reaction mixture was stirred cold for 15 minutes, and then was allowed to warm to ambient temperature overnight. The methanol was removed in vacuo. The remaining aqueous mixture was diluted with water (80 ml.) and washed with chloroform (2 x 30 ml.). The aqueous portion was acidified with concentrated HCl and extracted with ethyl acetate (3 x 65 ml.). The organic extract was washed with water and brine, dried ($MgSO_4$), and concentrated in vacuo to give 2.5 g. of crude product. Flash chromatography on 250 g. of Merck 9385 silica gel eluting with (10:1) toluene:acetone gave 1.35 g. of partially purified material. Three flash chromatographies on 125 g. columns of Merck 9385 silica gel eluting with (100:1:1) chloroform:methanol:acetic acid followed by a final flash chromatography on 100 g. of Merck 9385 silica gel eluting with (20:2:1) hexanes:ethyl acetate:acetic acid yielded 484 mg. of 4-[[2-(mercaptomethyl)-1-oxo-4-phenylbutyl]amino]benzoic acid as a white solid; m.p. 184 - 186°. TLC (silica gel; chloroform:methanol:acetic acid, 50:1:1) $R_f$ = 0.31.

| Anal. calc'd. for $C_{18}H_{19}NO_3S \cdot 0.26\ H_2O$: | | | | | |
|---|---|---|---|---|---|
| | C, 64.70; | H, 5.89; | N, 4.19; | S, 9.60; | SH, 9.90 |
| Found: | C, 64.51; | H, 5.93; | N, 4.38; | S, 9.66; | SH, 9.91. |

Example 14

4-[[2-(Mercaptomethyl)-1-oxo-3-[4-(phenylmethoxy)phenyl]propyl]amino]benzoic acid

a) 2-[(Acetylthio)methyl]-3-[4-(phenylmethoxy)phenyl]propanoic acid

A mixture of p-hydroxybenzaldehyde (91.8 g., 750 mmole), diethyl malonate (120 g., 750 mmole), acetic acid (20 ml.), and piperidine (6 ml.) in dry benzene (250 ml.) was heated under reflux for 7 hours and then allowed to cool to ambient temperature overnight. The solution was cooled in an acetone-dry ice bath and the solid that separated was filtered to give 138.1 g. of (p-hydroxybenzylidene) malonic acid, diethyl ester; m.p. 88 - 90°.

A solution of (p-hydroxybenzylidene) malonic acid, diethyl ester (26.4 g., 100 mmole) in absolute

ethanol (150 ml.) was treated with 10% palladium on carbon catalyst ( 1 g.) and shaken on a Parr apparatus pressurized with hydrogen for 2.5 hours. The catalyst was removed by filtration, and the filtrate was concentrated in vacuo. The identical procedure was repeated twice with 52.8 g. (200 mmole) and 58.9 g. (220 mmole) of (p-hydroxybenzylidene) malonic acid, diethyl ester to give a total of 88.9 g. of (p-hydroxybenzyl) malonic acid, diethyl ester as a solid; m.p. 47 - 48°.

A solution of (p-hydroxybenzyl) malonic acid, diethyl ester (13.3 g., 50 mmole) in 1N sodium hydroxide (50 ml.) and 95% ethanol (50 ml.) was treated under nitrogen with benzyl bromide (8.5 g., 50 mmole), and the reaction mixture was stirred at ambient temperature overnight. The ethanol was removed in vacuo. The resulting suspension was extracted twice with ether (200 ml.). The ether extract was washed with brine (100 ml.), dried ($MgSO_4$), and concentrated in vacuo to yield 7.7 g. of [p-(benzyloxy)benzyl] malonic acid, diethyl ester as a yellow oil. The aqueous layer was acidified and extracted with ether. This ether extract was dried ($MgSO_4$) and concentrated in vacuo to yield 4.0 g. of [p-(benzyloxy)benzyl] malonic acid, monoethyl ester; m.p. 70 - 80°.

A suspension of the above diethyl ester product (7.7 g., 22 mmole) and the monoethyl ester product (4.0 g., 12 mmole) in 10% sodium hydroxide (100 ml.) was heated under reflux for 5 hours. The solution was filtered, cooled and acidified with 20% HCl. The resulting white solid was collected to give 7.1 g. of [p-(benzyloxy)benzyl] malonic acid as a white crystalline solid; m.p. 149 - 151° (dec.).

A mixture of the above malonic acid product (94.6 g., 320 mmole), 40% aqueous dimethylamine (37.7 g., 330 mmole), and 37% aqueous formaldehyde (26.7 g., 330 mmole) in water (700 ml.) was stirred overnight at ambient temperature. The crystalline solid that separates was filtered to give 104.6 g. of [p-(benzyloxy)benzyl][(dimethylamino)methyl] malonic acid; m.p. 114 - 116° (dec.).

A solution of [p-(benzyloxy)benzyl][(dimethylamino)methyl] malonic acid (78 g., 220 mmole) in water (3 l.) was heated under reflux for one hour (gas evolves), and then was refrigerated overnight. The mixture was acidified with 10% potassium bisulfate and the resulting white precipitate was extracted into ether. The ether extract was washed with brine, dried ($MgSO_4$), and concentrated in vacuo to a solid residue. This solid was triturated with petroleum ether and filtered to give 41 g. of [p-(benzyloxy)benzyl] acrylic acid; m.p. 107 - 110°.

This acrylic acid (26.8 g., 100 mmole) was added portionwise to thiolacetic acid (50 ml.) at ambient temperature, resulting in a fine suspension. The mixture was warmed briefly at 50°, and the resulting clear solution was stirred at ambient temperature under nitrogen for 72 hours. The crystalline precipitate was filtered and triturated with hexane to give 29.8 g. of crude product; m.p. 117 - 120°. Recrystallization from cyclohexane yielded pure 2-[(acetylthio)methyl]-3-[4-(phenylmethoxy)phenyl]propanoic acid; m.p. 121 - 123° (dec.). TLC (silica gel; ethyl acetate) $R_f$ = 0.71.

| Anal. calc'd. for $C_{19}H_{20}O_4S$: | | | |
|---|---|---|---|
| | C, 66.26; | H, 5.85; | S, 9.31; |
| Found: | C, 66.32; | H, 5.90; | S, 8.83. |

b) 4-[[2-[(Acetylthio)methyl]-1-oxo-3-[4-(phenylmethoxy)phenyl]propyl]amino]benzoic acid, methyl ester

2-[(Acetylthio)methyl]-3-[4-(phenylmethoxy)phenyl]propanoic acid (2.0 g., 5.81 mmole) was dissolved in freshly distilled ether (15 ml.) and cooled to -5°. Oxalyl chloride (0.74 ml., 5.81 mmole) was added dropwise followed by N,N-dimethylformamide (4 drops). The ice bath was removed and the reaction was allowed to warm to room temperature as gas evolved. After stirring at room temperature for 2 hours, the reaction was a clear yellow solution with a small amount of gummy yellow precipitate. The solvent was removed in vacuo and the residue was chased with tetrahydrofuran. The resulting yellow oil was dissolved in dichloromethane (10 ml.), cooled to 0°, and added to a solution of 4-aminobenzoic acid, methyl ester (0.88 g., 5.81 mmole) and diisopropylethyl amine (1.01 ml., 5.81 mmole) in dry dichloromethane (20 ml.) at 0°. After 3 hours, the reaction was washed with saturated sodium bicarbonate, 1.0 M HCl, and saturated sodium chloride. The organic phase was dried ($MgSO_4$), filtered, and the solvent removed in vacuo to yield 2.5 g. of crude product as an orange foam. Purification by flash chromatography (200 g. of Whatman LPS - 1; 26% ethyl acetate, hexane) yielded 1.92 g. of 4-[[2-[(acetylthio)methyl]-1-oxo-3-[4-phenylmethoxy)-phenyl]propyl]amino]benzoic acid, methyl ester as an off-white solid. TLC (silica gel; 30% ethyl acetate, 70% hexane) $R_f$ = 0.18.

c) 4-[[2-(Mercaptomethyl)-1-oxo-3-[4-(phenylmethoxy)phenyl]propyl]amino]benzoic acid

The methyl ester product from part (b) (1.72 g., 3.6 mmole) was suspended in methanol (24 ml.) and degassed by bubbling argon through the mixture. The reaction was cooled to 0° and 1.0 M sodium hydroxide (11.0 ml., 11.0 mmole) was added. After 1 hour, solid remained and additional methanol (4 ml.) and sodium hydroxide (3.6 ml., 3.6 mmole) were added. After stirring for 2 hours, the reaction was still heterogeneous and freshly distilled tetrahydrofuran (48 ml.) was added at which time all the solid dissolved. After stirring for 5.5 hours, another aliquot of sodium hydroxide (3.6 ml., 3.6 mmole) was added. The reaction was stirred for 1.5 hours then concentrated in vacuo and the residue was dissolved in water (100 ml.). The solution was acidified to pH 1 with concentrated HCl and extracted with ethyl acetate. The organic extracts were combined, dried ($MgSO_4$), filtered, and the solvent removed. The off-white solid was purified by flash chromatography (140 g. of Whatman LPS - 1; 5% acetic acid, 25% ethyl acetate, 70% hexane) to yield 1.34 g. of partially purified product. This solid was repurified by flash chromatography (42 g. of Whatman LPS-1; 5% acetic acid, 18% ethyl acetate, 77% hexane) to give 90 mg. of 4-[[2-(mercaptomethyl)-1-oxo-3-[4-(phenylmethoxy)phenyl]propyl]amino]benzoic acid as a white solid; m.p. 189 - 191° (dec.). TLC (silica gel; ethyl acetate:acetic acid:hexane, 25:5:70) $R_f$ = 0.28.

| Anal., calc'd. for $C_{24}H_{23}NO_4S \cdot 2.92\ H_2O$: | | | | |
|---|---|---|---|---|
| | C, 60.79; | H, 6.13; | N, 2.95; | S, 6.76 |
| Found: | C, 60.50; | H, 5.77; | N, 3.18; | S, 6.46. |

## Example 15

4-[[2-[(Acetylthio)methyl]-1-oxo-3-phenylpropyl]amino]benzoic acid

A solution of 2-[(acetylthio)methyl]-3-phenylpropanoic acid (15.06 g., 63.2 mmole) in dry tetrahydrofuran (200 ml.) under nitrogen was treated dropwise with oxalyl chloride (8.02 g., 63.2 mmole). The resulting solution was treated with N,N-dimethylformamide (5 drops) and stirred at ambient temperature for one hour. The reaction mixture was concentrated to dryness in vacuo. The residue was twice concentrated from dry tetrahydrofuran (100 ml.), and then used without further purification.

A solution of 4-aminobenzoic acid (8.67 g., 63.2 mmole) in dry acetonitrile (125 ml.) was treated with bis(trimethylsilyl)trifluoroacetamide (38.8 g., 150 mmole, 2.4 equivalents), and the mixture was stirred at ambient temperature for 30 minutes. The reaction mixture was cooled in an ice-bath, treated dropwise with a solution of the above acid chloride (nominally 63.2 mmole) in dry acetonitrile (125 ml.), and then allowed to warm to ambient temperature overnight. The reaction mixture was concentrated in vacuo, and the residue was partitioned between 400 ml. each of ethyl acetate and water. The organic layer was extracted with 5% sodium bicarbonate (3 x 250 ml.). The aqueous extract was acidified to a pH of 1 with concentrated HCl and extracted with ethyl acetate (3 x 400 ml.). The organic extract was washed with brine, dried ($MgSO_4$), and then concentrated in vacuo. The original ethyl acetate layer from the bicarbonate extractions was combined with the final organic extract to give 34.0 g. of slightly yellow solid. Two recrystallizations from hot chloroform/hexane gave 19.85 g. of 4-[[2-[(acetylthio)methyl]-1-oxo-3-phenylpropyl]amino]benzoic acid as a white crystalline solid; m.p. 184 - 186°. TLC (silica gel; toluene:acetic acid, 10:1) $R_f$ = 0.28.

| Anal. calc'd. for $C_{19}H_{19}NO_4S$: | | | | |
|---|---|---|---|---|
| | C, 63.85; | H, 5.36; | N, 3.92; | S, 8.97 |
| Found: | C, 63.51; | H, 5.25; | N, 3.86; | S, 9.07. |

Example 16

4-[[2-[(Benzoylthio)methyl]-1-oxo-3-phenylpropyl]amino]benzoic acid

a) 2-[(Benzoylthio)methyl]-3-phenylpropanoic acid

A mixture of benzyl acrylic acid (13.7 g., 85 mmole) [prepared as described in Example 1(b)] and thiobenzoic acid (15 ml., 127 mmole) in dichloromethane (170 ml.) was stirred under argon at reflux temperature for 3 days, after which it was concentrated in vacuo. The residue was recrystallized twice from ether hexane to give 4.7 g. of 2-[(benzoylthio)methyl]-3-phenylpropanoic acid as a white solid; m.p. 99 - 102°.

| Anal. calc'd. for $C_{17}H_{16}O_2S$: | | | |
|---|---|---|---|
| | C, 67.98; | H, 5.37; | S, 10.67 |
| Found: | C, 67.89; | H, 5.34; | S, 10.70. |

b) 4-[[2-[(Benzoylthio)methyl]-1-oxo-3-phenylpropyl]amino]benzoic acid

A solution of 2-[(benzoylthio)methyl]-3- phenylpropanoic acid (874 mg., 2.91 mmole) in tetrahydrofuran (10 ml.) under nitrogen was treated with oxalyl chloride (369 mg., 2.91 mmole) and then one drop of dimethylformamide was added cautiously. The reaction mixture was stirred at ambient temperature for one hour, and then concentrated in vacuo. The residue was taken up in tetrahydrofuran (10 ml.) and concentrated in vacuo twice to give the desired acid chloride.

A solution of 4-aminobenzoic acid (399 mg., 2.91 mmole) in dry acetonitrile (10 ml.) under nitrogen was treated with bis(trimethylsilyl) trifluoroacetamide (1.8 g., 6.98 mmole, 2.4 equiv.), and the mixture was cooled in an ice bath and treated dropwise with the above acid chloride (2.91 mmole) in dry acetonitrile (10 ml.). The mixture was allowed to warm to ambient temperature overnight. The reaction mixture was concentrated in vacuo. The residue was partitioned between 20 ml. each of ethyl acetate and water. The organic layer was further extracted with 5% sodium bicarbonate (3 x 10 ml.). The combined bicarbonate extract was acidified to pH 1 with concentrated HCl and extracted with ethyl acetate (3 x 20 ml.). A 500 mg. amount of solid insoluble in ethyl acetate was filtered off and saved. The combined ethyl acetate extract was concentratd in vacuo, and the solid residue was twice triturated from methanol and filtered to give a 260 mg. portion of crude product. The solid was triturated with acetonitrile and filtered to give 190 mg. of solid. Some solid which precipitated out of the original ethyl acetate solution after the bicarbonate extractions was filtered to afford an additional 250 mg. of material. The 500 mg., 190 mg., and 250 mg. batches of solid were combined and recrystallized from hot acetonitrile to give 323 mg. of product as a white solid. A second 76 mg. crop was collected for a total yield of 399 mg. of 4-[[2-[(benzoylthio)methyl]-1-oxo-3-phenylpropyl]amino]benzoic acid as a white solid; m.p. 223 - 225°; $[\alpha]_D$ = +0.6° (c = 0.5, dimethylformamide). TLC (silica gel; toluene:acetic acid, 20:1) $R_f$ = 0.29.

| Anal. calc'd. for $C_{24}H_{21}NO_4S \cdot 0.15\ H_2O$: | | | | |
|---|---|---|---|---|
| | C, 68.27; | H, 5.09; | N, 3.32; | S, 7.59 |
| Found: | C, 68.09; | H, 4.90; | N, 3.50; | S, 7.59. |

Example 17

4-[[2-(Mercaptomethyl)-1-oxo-3-phenylpropyl]amino]benzoic acid

The product of Example 1 was also prepared as follows.

A solution of 4-[[2-[(acetylthio)methyl]-1-oxo-3-phenylpropyl]amino]benzoic acid (12.7 g., 35.53 mmole in water (90 ml.) and concentrated ammonia (50 ml.) was flushed with argon, and stirred stoppered at ambient temperature. After 30 minutes, the reaction mixture was washed with ethyl acetate (3 x 75 ml.). The aqueous layer was acidified to a pH of 1 with concentrated HCl, and extracted with ethyl acetate (3 x 75 ml.). The combined organic extract was washed with brine, dried ($MgSO_4$), and concentrated in vacuo to give 11.4 g. of crude product. This material was triturated with hexanes and filtered to give 10.6 g. of 4-[[2-(mercaptomethyl)-1-oxo-3-phenylpropyl]amino]benzoic acid as a white solid; m.p. 179-181°. TLC (silica gel; hexane:ethyl acetate: acetic acid, 12:7:1) $R_f$ = 0.46 (minor impurity at 0.23).

| Anal. calc'd. for $C_{17}H_{17}NO_3S$: | | | | |
|---|---|---|---|---|
| | C, 64.74; | H, 5.43; | N, 4.44; | S, 10.17 |
| Found: | C, 64.73; | H, 5.40; | N, 4.43; | S, 10.11. |

Example 18

(cis)-4-[[2-(Mercaptomethyl)-1-oxo-3-phenylpropyl]amino]cyclohexanecarboxylic acid

a) (cis)-4-Aminocyclohexanecarboxylic acid, methyl ester, monohydrochloride

Thionyl chloride (1.46 ml., 20 mmole) was added dropwise to a stirred suspension of (cis)-4-aminocyclohexanecarboxylic acid (1.43g., 10 mmole) [prepared as described by Villani et al., J. Org. Chem., Vol. 29. p. 2585 - 2587, 1964] in methanol (25 ml.) under argon, maintaining the reaction temperature at -5° to -10° during the addition. The resulting solution was allowed to warm to room temperature overnight. The solvent was then evaporated and the white residue was chased with methanol, then toluene, and then heated with acetonitrile (approximately 40 ml.). The crystals that separated upon cooling were filtered to give 1.74 g. of (cis)-4-aminocyclohexanecarboxylic acid, methyl ester, monohydrochloride as white needles; m.p. 185 - 187°. TLC (silica gel; n-butanol:acetic acid:water, 4:1:1) $R_f$ = 0.43.

| Anal. calc'd. for $C_8H_{16}NO_2Cl$: | | | | |
|---|---|---|---|---|
| | C, 49.61; | H, 8.33; | N, 7.23; | Cl. 18.30 |
| Found: | C, 49.76; | H, 8.42; | N, 7.29; | Cl. 17.91. |

b) (cis)-4-[[2-[(Acetylthio)methyl]-1-oxo-3-phenylpropyl]amino]cyclohexanecarboxylic acid, methyl ester

A solution of 3-acetylthio-2-benzylpropanoic acid (166 g., 7 mmole) in ether (14 ml.) under nitrogen was treated with oxalyl chloride (0.61 ml., 7 mmole) followed by a drop of dimethylformamide and the mixture was stirred at room temperature for one hour. The solvent was evaporated and the residue was chased twice with dry tetrahydrofuran (15 ml.) to give the desired acid chloride.

A suspension of (cis)-4-aminocyclohexanecarboxylic acid, methyl ester, monohydrochloride (1.35 g., 7 mmole) in dichloromethane (25 ml.) at -5° under nitrogen was treated concurrently with a solution of the above acid chloride (nominally 7 mmole) in dichloromethane (14 ml.) and diisopropylethylamine (2.44 ml., 14 mmole). After the addition was complete, the reaction mixture was stirred for an additional hour, and

then allowed to warm to room temperature overnight. The reaction mixture was concentrated and the residue was taken up in ethyl acetate (50 ml.). The mixture was filtered to remove diisopropylethylamine hydrochloride and the resulting solution was washed with 10% potassium bisulfate, water, 5% sodium bicarbonate, water and brine, then dried ($MgSO_4$) and evaporated to give 2.51 g. of residue. This residue was flash chromatographed on silica gel (Merck 9385, 250 g.) eluting with petroleum ether:acetone (5:1) to yield 2.30 g. of (cis)-4-[[2-[(acetylthio)methyl]-1-oxo-3-phenylpropyl]amino]cyclohexanecarboxylic acid, methyl ester as a white solid; m.p. 86 - 92°. TLC (silica gel; petroleum ether:acetone, 3:1) $R_f$ - 0.52.

| Anal. calc'd. for $C_{20}H_{27}NO_4S$: | | | | |
|---|---|---|---|---|
| | C, 63.63; | H, 7.21; | N, 3.71; | S, 8.49 |
| Found: | C, 63.57; | H, 7.39; | N, 3.81; | S, 8.81. |

c) (cis)-4-[[2-(Mercaptomethyl)-1-oxo-3-phenylpropyl]amino]cyclohexanecarboxylic acid

A solution of the methyl ester product from part (b) (2.28 g., 6.04 mmole) in methanol (18 ml.) under nitrogen was cooled in an ice bath and treated with 1N sodium hydroxide dropwise over 20 minutes. The mixture was allowed to warm to room temperature and stirred for 30 hours. The mixture was concentrated. The aqueous residue was diluted with water (50 ml.) and washed with chloroform (3 x 15 ml.). The aqueous portion was acidified to pH of 1 with concentrated HCl and extracted with ethyl acetate (3 x 30 ml.). The combined organic extract was washed with water and brine, dried ($MgSO_4$), and evaporated to give 1.9 g. of a white foamy solid. Flash chromatography on silica gel (Merck 9385, 200 g.) eluting with toluene:acetic acid (10:1) yielded 1.15 g. of (cis)-4-[[2-(mercaptomethyl)-1-oxo-3-phenylpropyl]amino]-cyclohexanecarboxylic acid as an amorphous white solid; m.p. 126 - 132°. TLC (silica gel; toluene:acetic acid, 5:1) $R_f$ = 0.39.

| Anal. calc'd. for $C_{17}H_{23}NO_3S \cdot 0.1\ H_2O$: | | | | | |
|---|---|---|---|---|---|
| | C, 63.17; | H, 7.23; | N, 4.33; | S, 9.92; | SH,10.23 |
| Found: | C, 63.49; | H, 7.38; | N, 4.40; | S,10.12; | SH, 9.84. |

In a similar manner, by employing (trans)-4-aminocyclohexanecarboxylic acid [prepared as described by Johnston et al., Jour. of Med. Chem., Vol. 20, p 279 - 290, 1977] in the above procedure one obtains (trans)-4-[[2-(mercaptomethyl)-1-oxo-3-phenylpropyl]amino]cyclohexanecarboxylic acid.

Also, by employing (cis)-3-aminocyclohexanecarboxylic acid and (trans)-3-aminocyclohexanecarboxylic acid (also disclosed by Johnston et al.) in the above procedure, one obtains (cis)-3-[[2-(mercaptomethyl)-1-oxo-3-phenylpropyl]amino]cyclohexanecarboxylic acid and (trans)-3-[[2-(mercaptomethyl)-1-oxo-3-phenylpropyl]amino]cyclohexanecarboxylic acid.

## Example 19

(cis)-4-[[3,3,3-Trifluoro-2-(mercaptomethyl)-1-oxopropyl]amino]cyclohexanecarboxylic acid

a) (cis)-4-[[3,3,3-Trifluoro-2-[(acetylthio)methyl]-1-oxopropyl]amino]cyclohexanecarboxylic acid

A suspension of (cis)-4-aminocyclohexanecarboxylic acid (2.15 g., 15 mmole) in dry acetonitrile (distilled from calcium hydride) under argon was cooled to 0° and bis(trimethylsilyl) trifluoroacetamide (8 ml., 30 mmole) was added. The reaction mixture was allowed to stir and warm to room temperature overnight. An additional 4 ml. of bis(trimethylsilyl)trifluoroacetamide was added. After an additional 4 hours

of stirring, dimethylformamide (6 ml.) was added to bring the remaining solid into solution. After 4 hours, almost all of the solid had dissolved. The mixture was cooled to 5° and a solution of 2-[(acetylthio)methyl]-3,3,3-trifluoropropanoyl chloride [3.52 g., 15 mmole, prepared as described in Example 10(a)] in acetonitrile (7 ml.) was added dropwise. The reaction mixture was allowed to warm to room temperature and stirred for 36 hours. The solvents were removed in vacuo. The residue was partitioned between water (50 ml.) and ethyl acetate (30 ml.) and the organic layer was separated. The aqueous layer was extracted twice more with ethyl acetate, and the combined ethyl extract was washed with brine, dried ($MgSO_4$) and evaporated to give 8.2 g. of a yellow residue. A portion of this crude product (5.85 g.) was triturated with ethyl acetate:hexane (1:2) and the white solid that separated was filtered and washed with ethyl acetate:hexane (1:2 and then 2:1) to give 0.9 g. of pure (cis)-4-[[3,3,3-trifluoro-2-[(acetylthio)methyl]-1-oxopropyl]amino]-cyclohexanecarboxylic acid as a white solid; m.p. 171 - 174°. TLC (silica gel; toluene:acetic acid, 10:1) $R_f$ = 0.20.

b) (cis)-4-[[3,3,3-Trifluoro-2-(mercaptomethyl)-1-oxopropyl]amino]cyclohexanecarboxylic acid

Treatment of the product from part (a) with concentrated ammonium hydroxide according to the procedure of Example 10(c) yields (cis)-4-[[3,3,3-trifluoro-2-(mercaptomethyl)-1-oxopropyl]amino]-cyclohexanecarboxylic acid.

## Examples 20 - 50

Following the procedures of Examples 1 - 19 the following additional examples within the scope of this invention can be obtained:

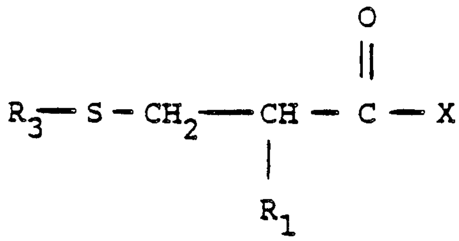

| Ex | $R_3$ | $R_1$ | X |
|---|---|---|---|
| 20 | H | $-C_2H_5$ | 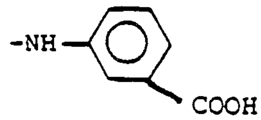 |
| 21 | H | $-CH(CH_3)_2$ | 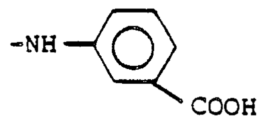 |
| 22 | H | 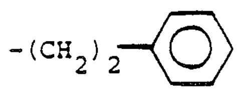 | 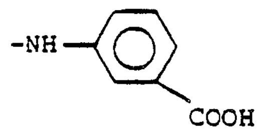 |
| 23 | H | 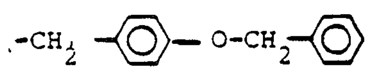 | 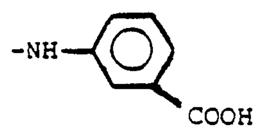 |
| 24 | H | $-C_3H_7$ | 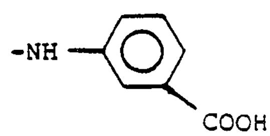 |

| Ex | $R_3$ | $R_1$ | X |
|---|---|---|---|
| 25 | H | 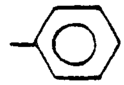 | 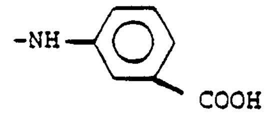 |
| 26 | H | 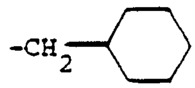 | 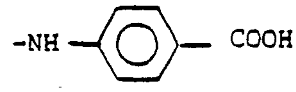 |
| 27 | H | 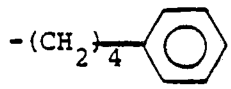 | 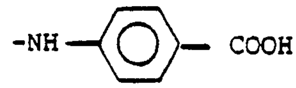 |
| 28 | H | 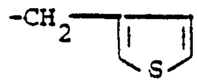 | 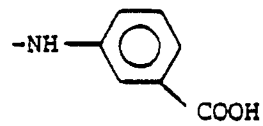 |
| 29 | H | 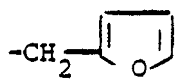 | 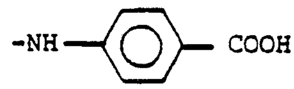 |
| 30 | H | 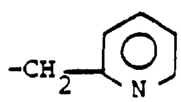 | 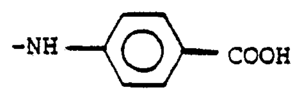 |
| 31 | H | 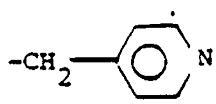 | 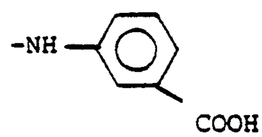 |
| 32 | H | 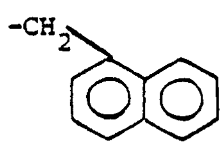 | 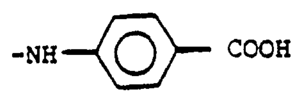 |

| Ex | $R_3$ | $R_1$ | X |
|---|---|---|---|
| 33 | H | 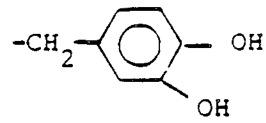 | 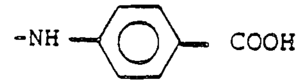 |
| 34 | H | 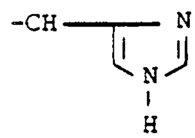 | 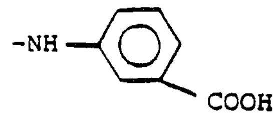 |
| 35 | H | 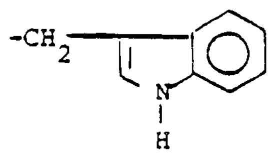 | 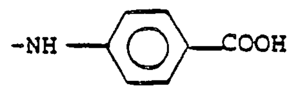 |
| 36 | H | $-(CH_2)_4-NH_2$ | 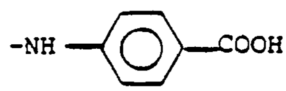 |
| 37 | H | $-(CH_2)_2-S-CH_3$ | 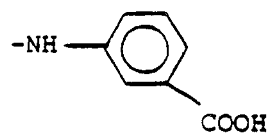 |
| 38 | H | 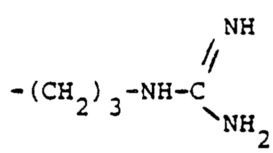 | 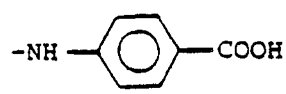 |
| 39 | H | 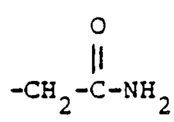 | 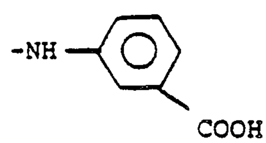 |

| Ex | $R_3$ | $R_1$ | X |
|---|---|---|---|
| 40 | 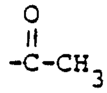 | $-CH-(CH_3)_2$ | 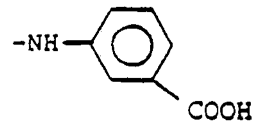 |
| 41 | 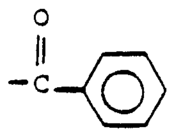 | 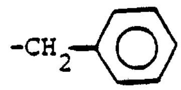 | 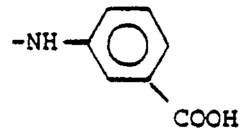 |
| 42 | 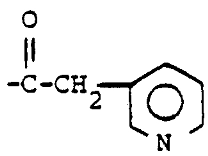 | $-C_2H_5$ | 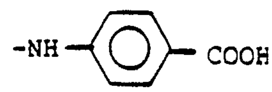 |
| 43 | 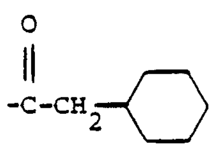 | 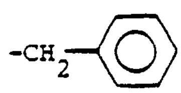 | 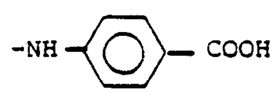 |
| 44 | 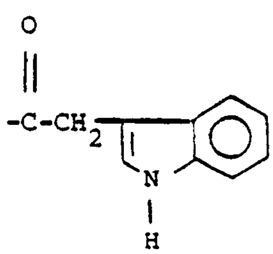 | 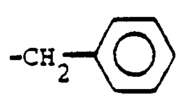 | 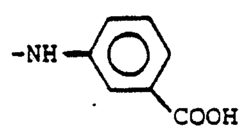 |

| Ex | $R_3$ | $R_1$ | X |
|---|---|---|---|
| 45 | H | $-CF_3$ | 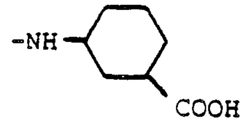 |
| 46 | H | $-CH(CH_3)_2$ | 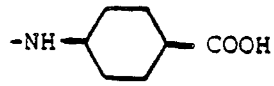 |
| 47 | H | $-CH_2CH(CH_3)_2$ | 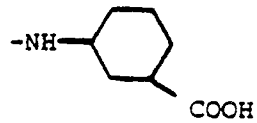 |
| 48 | H | $-C_2H_5$ | 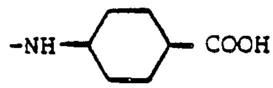 |
| 49 | H | $-(CH_2)_2-$ 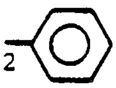 | 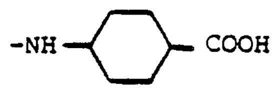 |
| 50 | H | 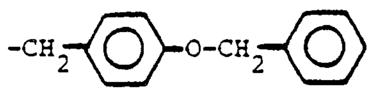 | 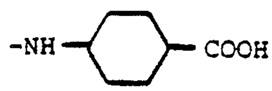 |

Example 51

1000 tablets each containing the following ingredients:

| | |
|---|---|
| 4-[[2-(Mercaptomethyl)-1-oxo-3-phenylpropyl]amino]benzoic acid | 100 mg. |
| Cornstarch | 50 mg. |
| Gelatin | 7.5 mg. |
| Avicel (microcrystalline cellulose) | 25 mg. |
| Magnesium stearate | 2.5 mg. |
| | 185 mg. |

are prepared from sufficient bulk quantities by mixing the product of Examples 1 or and cornstarch with an aqueous solution of the gelatin. The mixture is dried and ground to a powder. The Avicel and then the magnesium stearate are admixed with granulation. This mixture is then compressed in a tablet press to form 1000 tablets each containing 100 mg. of active ingredient. This same procedure can be employed to prepare tablets containing 50 mg. of active ingredient.

Similarly, tablets containing 50 mg. or 100 mg. of the product of any of Examples 2 to 16 and 18 to 50 can be prepared.

Example 52

Two piece #1 gelatin capsules are filled with a mixture of the following igredients:

| | |
|---|---|
| 3-[[2-(Mercaptomethyl)-1-oxo-3-phenylpropyl]amino]benzoic acid | 100 mg. |
| Magnesium stearate | 7 mg. |
| Lactose | 193 mg. |
| | 300 mg. |

In a similar manner, capsules containing 100 mg. of the product of any of Examples 1 to 6, 8 to 16, and 18 to 50 can be prepared.

Example 53

An injectable solution is prepared as follows:

| | |
|---|---|
| 3-[[3,3,3-Trifluoro-2-(mercaptomethyl)-1-oxopropyl]amino]benzoic acid | 500 g. |
| Methyl paraben | 5 g. |
| Propyl paraben | 1 g. |
| Sodium chloride | 25 g. |
| Water for injection | 5 l. |

The active substance, preservatives and sodium chloride are dissolved in 3 liters of water for injection and then the volume is brought up to 5 liters. The solution is filtered through a sterile filter and aseptically filled into presterilized vials with rubber closures. Each vial contains 5 ml. of solution in a concentration of 100 mg. of active ingredient per ml. of solution for injection.

In a similar manner, an injectable solution containing 100 mg. of active ingredient per ml. of solution can be prepared for the product of any of Examples 1 to 10, 12 to 16, and 18 to 50.

**Claims**

1. A compound of the formula

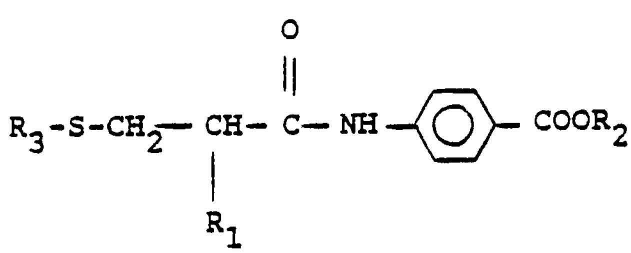

or a pharmaceutically acceptable salt thereof wherein:
$R_1$ is hydrogen, straight or branched chain lower alkyl of 2 to 7 carbons, halo substituted lower alkyl,

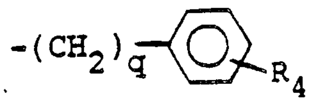,

$-(CH_2)_r$-cycloalkyl, $-(CH_2)_r$-(α-naphthyl), $-(CH_2)_r$-(β-naphthyl),

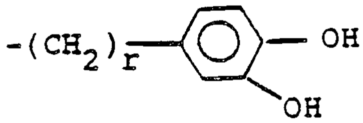, 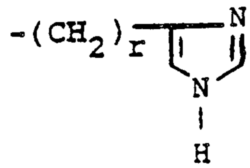,

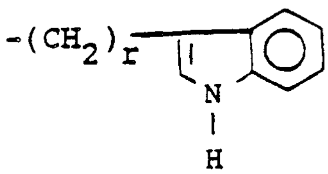,

$-(CH_2)_r-NH_2$, $-(CH_2)_r-SH$, $-(CH_2)_r$-S-lower alkyl,

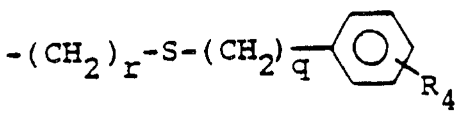,

$-(CH_2)_r-OH$, $-(CH_2)_r$-O-lower alkyl,

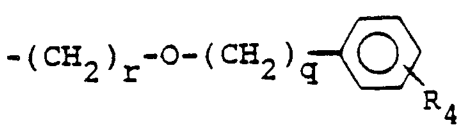, 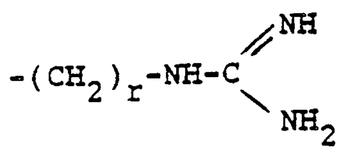,

or

$-(CH_2)_r-\overset{O}{\overset{\|}{C}}-NH_2$;

$R_2$ is hydrogen, lower alkyl, benzyl, benzhydryl, a pharmaceutically acceptable salt forming ion, or

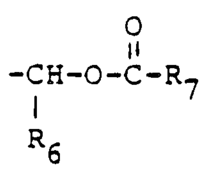;

$R_3$ is hydrogen or

$R_5-\overset{O}{\overset{\|}{C}}-$;

$R_5$ is lower alkyl,

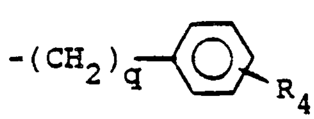,

$-(CH_2)_q$-($\alpha$-naphthyl), $-(CH_2)_q$-($\beta$-naphthyl),

$-(CH_2)_q$-cycloalkyl, 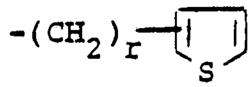 , 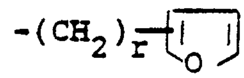 ,

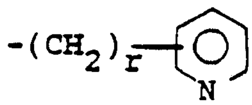 , 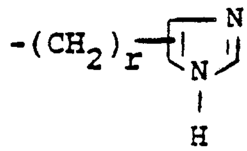 , or

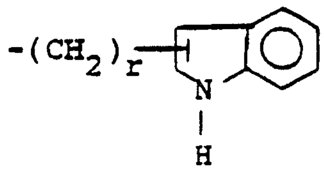 ;

$R_4$ is hydrogen, lower alkyl of 1 to 4 carbons, lower alkoxy of 1 to 4 carbons, lower alkylthio of 1 to 4 carbons, halo, hydroxy, $CF_3$, phenyl,

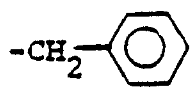 , or 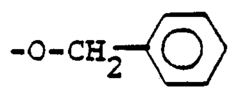 ;

$R_6$ is hydrogen, lower alkyl, cycloalkyl, or phenyl;
$R_7$ is hydrogen, lower alkyl, lower alkoxy or phenyl;
r is an integer from 1 to 4; and
q is zero or an integer from 1 to 7.

2. A compound of Claim 1 wherein:
$R_1$ is straight or branched chain alkyl of 2 to 4 carbons,

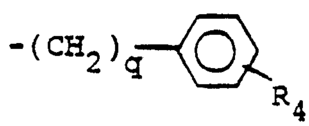 ,

or trifluoromethyl;
$R_2$ is hydrogen, methyl, or an alkali metal salt ion;
$R_3$ is hydrogen,

$-\overset{O}{\overset{\|}{C}}-CH_3$ or

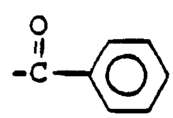 ;

$R_4$ is hydrogen or

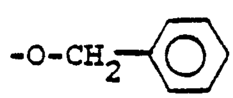 ;

q is zero or an integer from 1 to 4.

3. A compound of Claim 2 wherein:

$R_2$ is hydrogen; and
$R_3$ is hydrogen.

4. A compound of Claim 3 wherein:

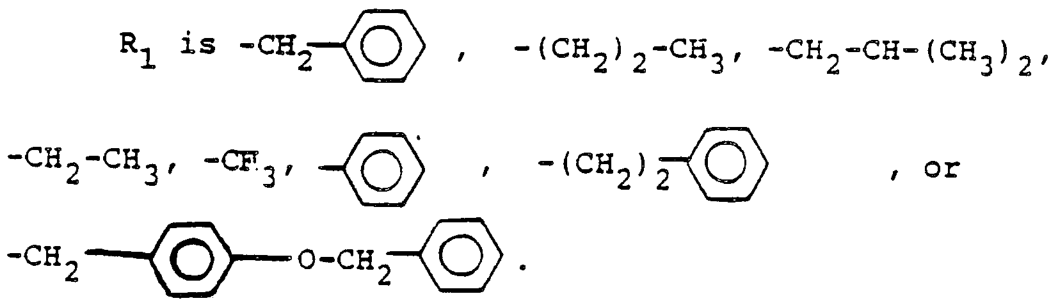

5. A compound of Claim 2 wherein:

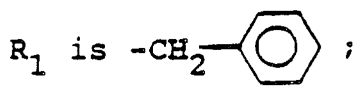

$R_2$ is hydrogen; and
$R_3$ is

$-\overset{\overset{C}{\|}}{C}-CH_3$ or

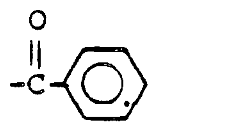

6. A compound of the formula

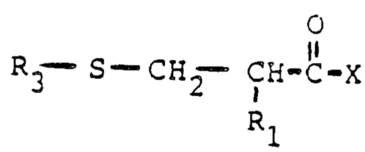

or a pharmaceutically acceptable salt thereof wherein:

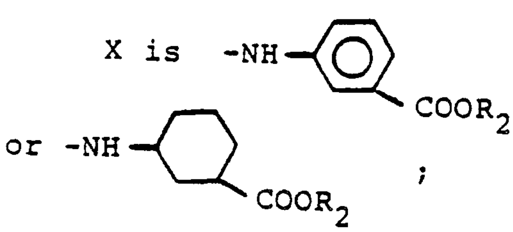

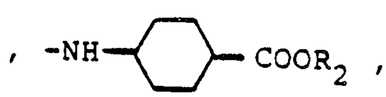

$R_1$ is hydrogen, lower alkyl, halo substituted lower alkyl,

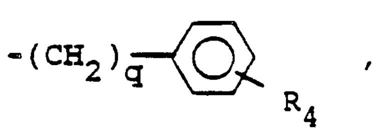

$-(CH_2)_r$-cycloalkyl, $-(CH_2)_r$-(α-naphthyl), $-(CH_2)_r$-(β-naphthyl),

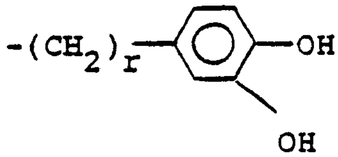 , 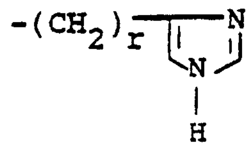 ,

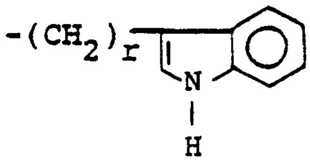

$-(CH_2)_r-NH_2$, $-(CH_2)_r-SH$, $-(CH_2)_r$-S-lower alkyl,

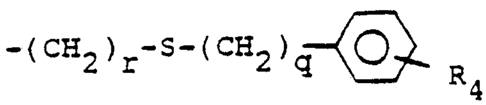 ,

$-(CH_2)_r-OH$, $-(CH_2)_r$-O-lower alkyl,

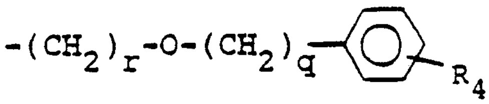 , 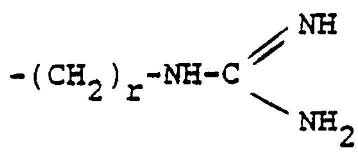 ,

or

$-(CH_2)_r-\overset{O}{\overset{\|}{C}}-NH_2$ ;

$R_2$ is hydrogen, lower alkyl, benzyl, benzhydryl, a pharmaceutically acceptable salt forming ion, or

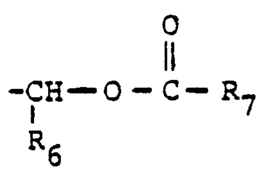 ;

$R_3$ is hydrogen or

$R_5-\overset{O}{\overset{\|}{C}}-$ ;

$R_5$ is lower alkyl,

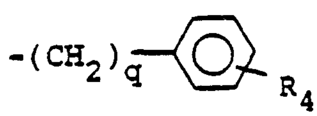 ,

$-(CH_2)_q$-(α-naphthyl), $-(CH_2)_q$-(β-naphthyl),

$-(CH_2)_q$-cycloalkyl, 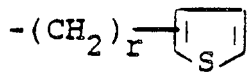 ,

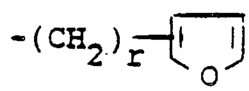 , 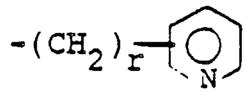 ,

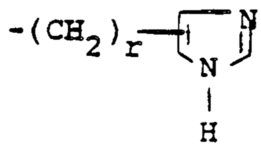 , or 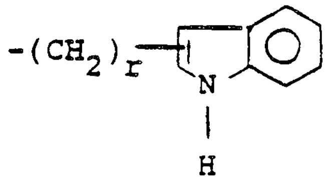 ;

$R_4$ is hydrogen, lower alkyl of 1 to 4 carbons, lower alkoxy of 1 to 4 carbons, lower alkylthio of 1 to 4 carbons, halo, hydroxy, $CF_3$, phenyl,

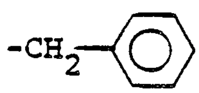 , or 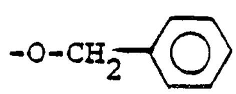 ;

$R_6$ is hydrogen, lower alkyl, cycloalkyl, or phenyl;
$R_7$ is hydrogen, lower alkyl, lower alkoxy, or phenyl;
r is an integer from 1 to 4; and
q is zero or an integer from 1 to 7.

7. A compound of Claim 6 wherein:
R· is straight or branched chain alkyl of 2 to 4 carbons,

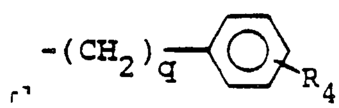

or trifluoromethyl;
$R_2$ is hydrogen, methyl, or an alkali metal salt ion;
$R_3$ is hydrogen,

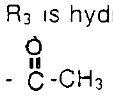 or

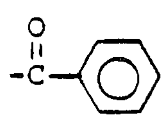 ;

$R_4$ is hydrogen or

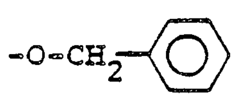 ;

q is zero or an integer from 1 to 4.

8. A compound of Claim Claim 7 wherein:
X is

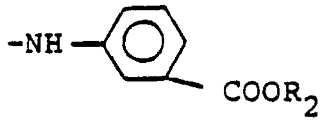 .

9. A compound of Claim 8 wherein:
$R_2$ is hydrogen; and
$R_3$ is hydrogen.

10. A compound of Claim 9 wherein:
$R_1$ is

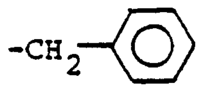 ,

$-CH_2-CH-(CH_3)_2$ , or $CF_3$ .

11. A compound of Claim 6 wherein:
X is

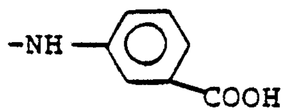 ;

$R_1$ is methyl; and
$R_3$ is hydrogen.

12. A compound of Claim 7 wherein:
X is

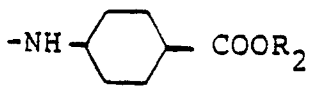 .

13. A compound of Claim 12 wherein:
$R_2$ is hydrogen; and
$R_3$ is hydrogen.

14. A compound of Claim 13 wherein:
$R_1$ is

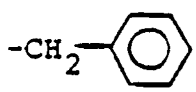

or $CF_3$ .

15. A compound of Claim 7 wherein

X is 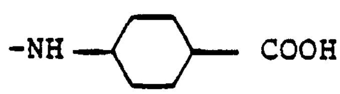 ,

$R_1$ is 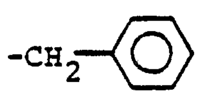 ,

and
$R_3$ is

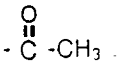

16. A compound of Claim 7 wherein
X is

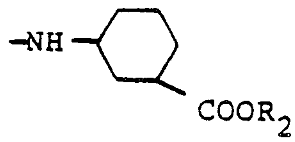

17. A pharmaceutical composition useful for reducing blood pressure and producing diuresis and natriuresis as well as treating congestive heart failure, pain and/or diarrhea in a mammalian host comprising a pharmaceutically acceptable carrier and an endopeptidase inhibiting compound of the formula

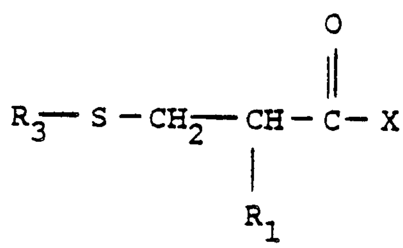

or a pharmaceutically acceptable salt thereof wherein:

X is

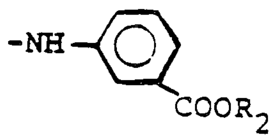 , 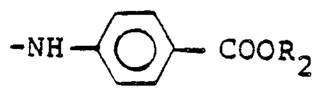 ,

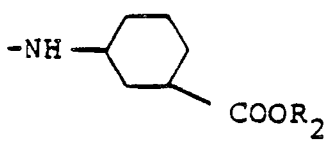 , or 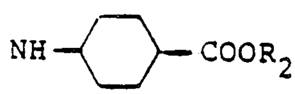 ;

$R_1$ is hydrogen, lower alkyl, halo substituted lower alkyl,

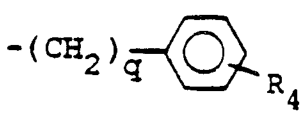 ,

$-(CH_2)_r$-cycloalkyl, $-(CH_2)_r$-($\alpha$-naphthyl), $-(CH_2)_r$-($\beta$-naphthyl),

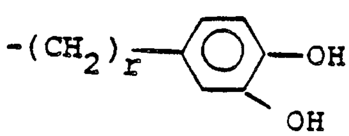 , 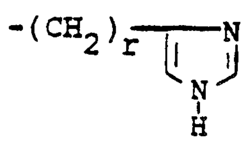 ,

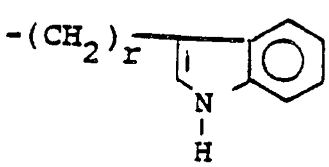

$-(CH_2)_r-NH_2$, $-(CH_2)_r-SH$, $-(CH_2)$-S-lower alkyl,

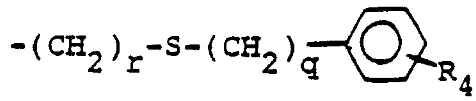

,

$-(CH_2)_r-OH$, $-(CH_2)_r$-O-lower alkyl,

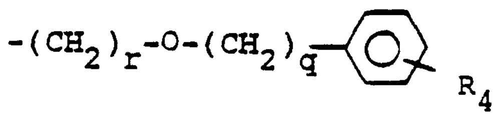

,

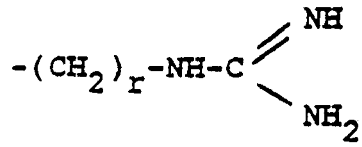

,

or

$-(CH_2)_r-\overset{O}{\overset{\|}{C}}-NH_2$;

$R_2$ is hydrogen, lower alkyl, benzyl, benzhydryl, a pharmaceutically acceptable salt forming ion, or

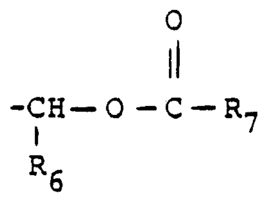

;

$R_3$ is hydrogen or

$R_5-\overset{O}{\overset{\|}{C}}-$ ;

$R_5$ is lower alkyl,

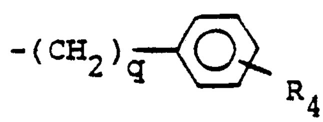

,

$-(CH_2)_q$-($\alpha$-naphthyl), $-(CH_2)_q$-($\beta$naphthyl),

$-(CH_2)_q$-cycloalkyl,

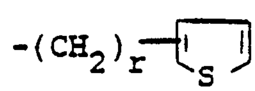

,

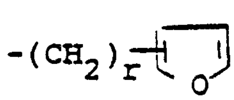

,

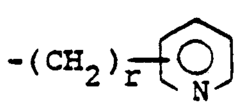

,

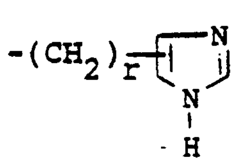

, or

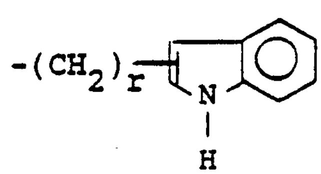

;

$R_4$ is hydrogen, lower alkyl of 1 to 4 carbons, lower alkoxy of 1 to 4 carbons, lower alkylthio of 1 to 4

carbons, halo, hydroxy, $CF_3$, phenyl,

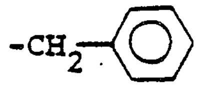 , or 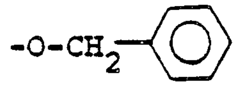 ;

$R_6$ is hydrogen, lower alkyl, cycloalkyl, or phenyl;
$R_7$ is hydrogen, lower alkyl, lower alkoxy, or phenyl;
r is an integer from 1 to 4; and
q is zero or an integer from 1 to 7.

European Patent Office

# EUROPEAN SEARCH REPORT

Application Number

EP 89 11 7683

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,Y | US-A-4 132 802 (T.A. MARTIN)<br>* Column 1, line 45 - column 2, line 33; column 6, lines 61,62 *<br>--- | 1,6,11 | C 07 C 323/60<br>C 07 C 327/32<br>A 61 K 31/195 |
| Y | CHEMICAL ABSTRACTS, vol. 67, no. 5, 31th July 1967, page 2036, abstract no. 21570j, Columbus, Ohio, US; G.M. AIRAPETYAN et al.: "Preparation of some derivatives of beta-mercaptopropionic acid, cystamine, and cycteamine and study of their radioprotective activity", & IZV. AKAD. NAUK SSSR, SER. KHIM. 1967(2),334-41<br>* Page 2036, column 2, line 18 *<br>--- | 1-3 | |
| A | BE-A- 890 948<br>* Claim 1 *<br>--- | 1-3,6-11 | |
| A | EP-A-0 115 997 (ROUSSEL-UCLAF)<br>* Page 15, example 43; claim 1 *<br>----- | | |

TECHNICAL FIELDS SEARCHED (Int. Cl.5)

C 07 C 323/00
C 07 C 327/00

The present search report has been drawn up for all claims

| Place of search | Date of completion of the search | Examiner |
|---|---|---|
| THE HAGUE | 06-12-1989 | ZAROKOSTAS K. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)